# EUROPÄISCHE PATENTSCHRIFT

(11) **EP 2 820 001 B1**
(45) Veröffentlichungstag und Bekanntmachung des Hinweises auf die Patenterteilung: **20.04.2016**
(21) Anmeldenummer: 13709804.2
(22) Anmeldetag: 28.02.2013
(51) Int. Cl.: C07D 239/84, A61K 31/517, A61P 31/22, C07D 239/74

(54) **NATRIUM- UND CALCIUMSALZE EINES DIHYDROCHINAZOLINDERIVATES UND IHRE VERWENDUNG ALS ANTIVIRALE MITTEL**
SODIUM AND CALCIUM SALTS OF A DIHYDROQUINAZOLINE DERIVATE AND THEIR USE AS ANTIVIRAL AGENTS
SELS DE SODIUM ET DE CALCIUM D'UN DÉRIVÉ DE DIHYDROQUINAZOLINE ET LEUR UTILISATION EN TANT QU'AGENTS ANTIVIRAUX

(30) Priorität: 29.02.2012 DE 102012101659
(43) Veröffentlichungstag der Anmeldung: 07.01.2015
(73) Patentinhaber: AiCuris Anti-infective Cures GmbH, 42117 Wuppertal (DE)
(72) Erfinder: GRUNENBERG, Alfons, 42115 Wuppertal (DE); BERWE, Mathias, 45549 Sprockhövel (DE); KEIL, Birgit, 40231 Düsseldorf (DE); ARET, Edwin, NL-1333 CJ Almere (NL); PAULUS, Kerstin, 40882 Ratingen (DE); SCHWAB, Wilfried, 14542 Werder (Havel) (DE)
(74) Vertreter: Kilger, Ute
(86) Internationale Anmeldenummer: PCT/EP2013/054115
(87) Internationale Veröffentlichungsnummer: WO 2013/127971

(56) Entgegenhaltungen:
- WO-A1-2004/096778

## Beschreibung

Die vorliegende Erfindung betrifft Salze von {8-Fluor-2-[4-(3-methoxyphenyl)piperazin-1-yl]-3-[2-methoxy-5-(trifluormethyl)phenyl]-3,4-dihydrochinazolin-4-y}essigsäure und deren Solvate.

Die Erfindung betrifft ferner Verfahren zu ihrer Herstellung, diese Verbindungen zur Verwendung zur Behandlung und/oder Prophylaxe von Krankheiten, insbesondere von Virusinfektionen sowie ihre Verwendung zur Herstellung von Arzneimitteln zur Behandlung und/oder Prophylaxe von Virusinfektionen insbesondere zur Behandlung und/oder Prophylaxe von Infektionen mit Cytomegalieviren oder einem anderen Vertreter der Gruppe der Herpes viridae. {8- Fluor-2-[4-(3-methoxyphenyl)piperazin-1-yl]-3-[2-methoxy-5-(trifluromethyl)phenyl]-3,4-dihydrochinazolin-4-yl}essigsäure ist z.B. aus der WO 2004/096778 bekannt und wurde von der Anmelderin als aussichtsreicher Kandidat für eine antiviral wirksame Substanz, insbesondere zur Bekämpfung von Infektionen mit dem humanen Cytomegalievirus (HCMV) entwickelt. Bei der Entwicklung hat es sich allerdings als äußerst kompliziert erwiesen, die Verbindung, sei es als Zwitterion, sei es in Salzform, in kristalliner Form zu erhalten und bis jetzt erfolgte die Entwicklung unter Verwendung des Zwitterions in amorpher Form. Die Verwendung amorpher Substanzen zur Herstellung von Arzneimitteln ist allerdings zum einen im Hinblick darauf, dass es häufig schwierig ist, bei amorphen Substanzen eine gleichmäßige Reinheit zu garantieren, sowie auch im Hinblick darauf, dass es bei amorphen Substanzen häufig schwierig ist, gleichbleibende pharmakologische Parameter wie beispielsweise eine gleichbleibende Bioverfügbarkeit zu garantieren, unerwünscht.

Es ist somit eine Aufgabe der Erfindung, Salze von {8-Fluor-2-[4-(3-methoxyphenyl)piperazin-1-yl]-3-[2-methoxy-5-(trifluormethyl)phenyl]-3,4-dihydro-chinazolin-4-yl}essigsäure zu beschreiben, mit denen kristalline Produkte erhalten werden können. Damit die Salze bei der Entwicklung von Medikamenten sinnvoll eingesetzt werden können, müssen diese ferner über lange Zeit lagerstabil sein. Schließlich sollten die kristallinen Verbindungen auch in wässrigem Medium und insbesondere bei physiologischem pH gut löslich sein.

Ferner weisen die erfindungsgemäßen Salze eine hohe Reinheit auf.

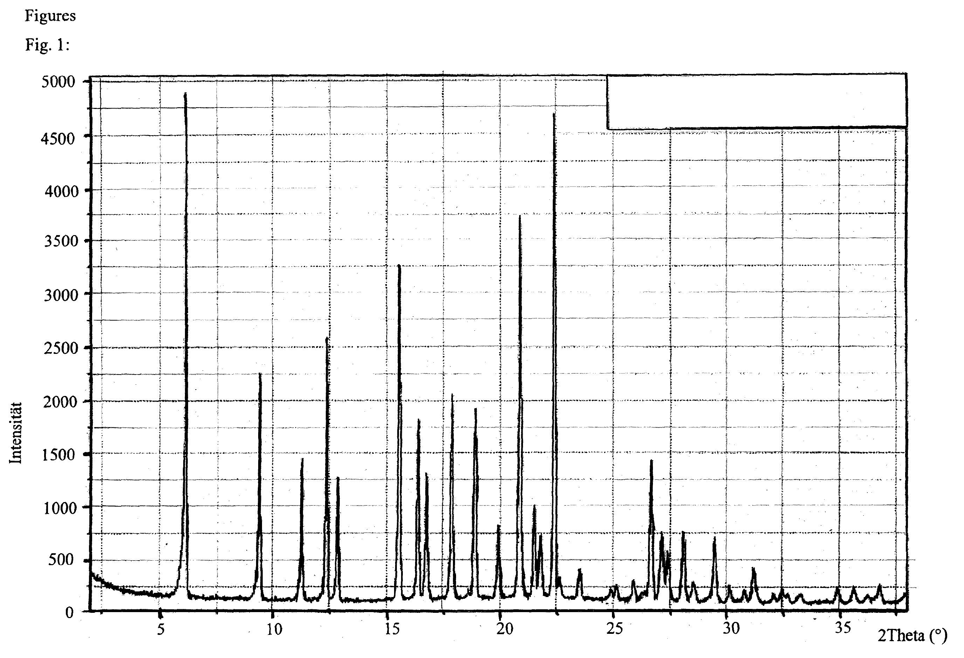

Der Begriff "kristallines Produkt" im Kontext der vorliegenden Erfindung bezeichnet S(+)-{8-Fluor-2-[4-(3-methoxyphenyl}piperazin-1-yl]-3-[2-methoxy-5-(trifluormethyl)phenyl]-3,4-dihydrochinazolin-4-yl}essigsäure Natrium-Salze und S(+)-{8-Fluor-2-[4-(3-methoxyphenyl)piperazin-1-yl]-3-[2-methoxy-5-(trifluormethyl)phenyl]-3,4-dihydrochinazolin-4-yl}essigsäure Calcium-Salze, welche unter Röntgendiffraktions-Analyse das charakteristische Peak-Muster wie in den entsprechenden Figuren 1 - 3 dargestellt oder ein ähnliches Peak-Muster aufweisen.

In dem Kontext der Erfindung bezeichnet der Begriff "lagerstabil" für die erfindungsgemäßen Salze bei 25°C, einen Mindestanteil an {8-Fluor-2-[4-(3-methoxyphenyl)piperazin-1-yl]-3-[2-methoxy-5-(trifluormethyl)phenyl]-3,4-dihydrochinazolin-4-yl}essigsäure von > 90%, vorzugsweise > 95%, am meisten bevorzugt > 99% für mindestens zwei, vorzugsweise mindestens drei, noch mehr bevorzugt mindestens sechs, am meisten bevorzugt mindestens 12 Monate Lagerung, wenn mittels einer der HPLC Methoden 1 - 3 die besagten Salze gemessen werden. Diese besagte Lagerstabilität der Salze wird im Rahmen der Erfindung als ausreichend angesehen.

Überraschenderweise wurde nun herausgefunden, dass {8-Fluor-2-[4-(3-methoxyphenyl)piperazin-1-yl]-3-[2-methoxy-5-(trifluormethyl)phenyl]-3,4-dihydrochinazolin-4-yl}essigsäure mit Natrium- und Calciumkationen gut definierte kristalline Salze bildet. Es hat sich ferner gezeigt, dass diese Salze in wässrigem Medium, insbesondere bei physiologischem pH, gut löslich sowie auch gut lagerstabil sind.

Ferner zeichnen sich die erfindungsgemäß erhaltenen kristallinen Natrium- und Calcium-Salze der {8-Fluor-2-[4-(3-methoxyphenyl)piperazin-1-yl]-3-[2-methoxy-5-(trifluormethyl)phenyl]-3,4-dihydrochinazolin-4-yl}essigsäure durch hohe Reinheit aus.

Die Begriffe "hohe Reinheit, Reinheit und rein" im Zusammenhang mit den erfindungsgemäßen S(+)-{8-Fluor-2-[4-(3-methoxyphenyl)piperazin-1-yl]-3-[2-methoxy-5-(trifluormethyl)phenyl]-3,4-dihydrochinazolin-4-yl}essigsäure Natrium-Salze und S(+)-{8-Fluor-2-[4-(3-methoxyphenyl)piperazin-1-yl]-3-[2-methoxy-5-(trifluormethyl)-phenyl]-3,4-dihydrochinazolin-4-yl}essigsäure Calcium-Salze bezeichnet dessen Vorhandensein als Stoff in einem Stoffgemisch mit einem < 0,1 %, vorzugsweise < 0,08%, weiter vorzugsweise < 0,05%, am meisten bevorzugt < 0,01% Gesamtanteil dessen bekannter Verunreinigungen Di-p-Toluoyl-D-Weinsäure, und/oder S-Chinazolylpiperazin, und/oder Chinazolin Ethylester und/oder Chinazolyldipiperazin, und/oder dessen unspezifische Verunreinigungen, wenn mittels HPLC gemäß Ausführungsbeispiel F) gemessen wird.

Gegenstand der Erfindung sind somit die kristallinen Natriumsalze und kristallinen Calciumsalze von {8-Fluor-2-[4-(3-methoxyphenyl)piperazin-1-yl]-3-[2-methoxy-5-(trifluormethyl)phenyl]-3,4-dihydrochinazolin-4-yl}essigsäure, sowie deren Solvate.

Natrium und Calciumsalze von {8-Fluor-2-[4-(3-methoxyphenyl)piperazin-1-yl]-3-[2-methoxy-5-(trifluormethyl)phenyl]-3,4-dihydrochinazolin-4-yl}essigsäure sind im Rahmen der Erfindung Addukte einer Reaktion der {8-Fluor-2-[4-(3-methoxyphenyl)piperazin-1-yl]-3-[2-methoxy-5-(trifluormethyl)phenyl]-3,4-dihydrochinazolin-4-yl}essigsäure mit starken Natrium- oder Calciumbasen, insbesondere Natriumhydroxid oder Calciumhydroxid. Die {8-Fluor-2-[4-(3-methoxyphenyl)piperazin-1-yl]-3-[2-methoxy-5-(trifluormethyl)phenyl]-3,4-dihydrochinazolin-4-yl}essigsäure und die Natrium- bzw. Calciumgegenionen können hierbei in jedem Verhältnis vorliegen. Bevorzugt ist das Verhältnis dabei ganzzahlig (z.B. 1:1, 1:2, 1:3, 3:1, 2:1). Die Salze können dabei durch eine direkte Umsetzung der {8-Fluor-2-[4-(3-methoxyphenyl)piperazin-1-yl]-3-[2-methoxy-5-(trifluormethyl)phenyl]-3,4-dihydro-chinazolin-4-yl}essigsäure mit Natrium- oder Calciumbasen oder durch Herstellung eines anderen basischen Salzes der {8-Fluor-2-[4-(3-methoxyphenyl)piperazin-1-yl]-3-[2-methoxy-5-(trifluormethyl)phenyl]-3,4-dihydrochinazolin-4-yl}essigsäure gefolgt von einem Austausch des Gegenions hergestellt werden.

Als Solvate werden im Rahmen der Erfindung solche Formen der Salze von {8-Fluor-2-[4-(3-methoxyphenyl)piperazin-1-yl]-3-[2-methoxy-5-(trifluormethyl)phenyl]-3,4-dihydrochinazolin-4-yl}essigsäure bezeichnet, welche durch Koordination mit Lösungsmittelmolekülen einen Komplex bilden. Hydrate sind eine spezielle Form der Solvate, bei denen die Koordination mit Wasser erfolgt.

Bevorzugt im Rahmen der vorliegenden Erfindung ist das Monocalciumsalz von {8-Fluor-2-[4-(3-methoxyphenyl)piperazin-1-yl]-3-[2-methoxy-5-(trifluormethyl)phenyl]-3,4-dihydrochinazolin-4-yl}essigsäure und insbesondere das 2,5-Hydrat und das 3,5-Hydrat des Monocalciumsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure. Ferner bevorzugt ist im Rahmen der Erfindung das Mononatriumsalz von {8-Fluor-2-[4-(3-methoxyphenyl)piperazin-1-yl]-3-[2-methoxy-5-(trifluormethyl)phenyl]-3,4-dihydrochinazolin-4-yl}essigsäure und insbesondere das 3-Hydrat des Mononatriumsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure.

Bevorzugt im Rahmen der Erfindung ist ferner ein 2,5-Hydrat des Monocalciumsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure, das im Pulver-XRD-Diffraktogramm charakteristische Peaks bei etwa 6.1, 9.2 und 15.5 Grad 2Theta zeigt.

Bevorzugt im Rahmen der Erfindung ist ferner ein 3,5-Hydrat des Monocalciumsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure, das im Pulver-XRD-Diffraktogramm charakteristische Peaks bei etwa 6.2, 12.4 und 22.4 Grad 2Theta zeigt.

Bevorzugt im Rahmen der Erfindung ist ferner ein 3-Hydrat des Mononatriumsalzes von {8-Fluor-2-[4-(3-methoxyphenyl)piperazin-1-yl]-3-[2-methoxy-5-(trifluormethyl)-phenyl]-3,4-dihydrochinazolin-4-yl]essigsäure, das im Pulver-XRD-Diffraktogramm charakteristische Peaks bei etwa 6.2, 20.9 und 22.4 Grad 2Theta zeigt.

Wie es einem Fachmann ohne Weiteres ersichtlich ist, weist {8-Fluor-2-[4-(3-methoxyphenyl)piperazin-1-yl]-3-[2-methoxy-5-(trifluormethyl)phenyl]-3,4-dihydrochinazolin-4-yl}essigsäure am Kohlenstoff in 4-Position des Dihydrochinazolinrings ein Stereozentrum auf. Im Rahmen der vorliegenden Erfindung ist es besonders bevorzugt, wenn dieser Kohlenstoff die S-Konfiguration aufweist.

Die erfindungsgemäßen Salze werden im Allgemeinen durch Umsetzen von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder eines Säuresalzes davon mit einer Natrium- oder Calciumbase, insbesondere Natriumhydroxid oder Calciumhydroxid, in einem Lösungsmittel hergestellt.

Es ist ferner möglich ein Basensalz von {8-Fluor-2-[4-(3-methoxyphenyl)piperazin-1-yl]-3-[2-methoxy-5-(trifluormethyl)phenyl]-3,4-dihydrochinazolin-4-yl}essigsäure, bei dem es sich nicht um eine Natrium- oder Calciumsalz handelt, mit einer Quelle für Natrium- oder Calciumkationen in einem Lösungsmittel umzusetzen.

Insbesondere wird bei den oben erwähnten Umsetzungen als Lösungsmittel eine Mischung aus zumindest einem Di-(C₁-C₄)-alkylether und zumindest einem (C₁-C₄)-Alkohol eingesetzt.

Gegenstand der Erfindung ist somit auch ein Verfahren zur Herstellung eines Natrium- oder Calciumsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure mit den folgenden Schritten:
a.) Lösen von {8-Fluor-2-[4-(3-methoxyphenyl)piperazin-1-yl]-3-[2-methoxy-5-(trifluormethyl)phenyl]-3,4-dihydrochinazolin-4-yl}-essigsäure oder eines Solvats davon in einer Mischung aus zumindest einem Di-(C₁-C₄)-alkylether und zumindest einem (C₁-C₄)-Alkohol, ggf. unter Erwärmen.
b.) Zugabe von NaOH oder Ca(OH)₂ zu der in Schritt a.) erhaltenen Lösung,
c.) Entfernen eines Teil des Lösungsmittels aus der in Schritt b.) erhaltenen Lösung und ggf. Animpfen mit geeigneten Impfkristallen, um die Kristallisation des Salzes bzw. eines Solvats des Salzes auszulösen,
d.) Abtrennen des in Schritt c.) auskristallisierten Salzes oder Solvats davon,
e.) Ggf Rühren des in Schritt d.) erhaltenen Salzes oder Solvats in einem geeigneten Lösungsmittel, vorzugsweise in einer Mischung aus Wasser und zumindest einem (C₁-C₄)-Alkohol um ein gewünschtes Solvat zu erhalten, und
f.) Trocknen des in Schritt d.) oder e.) erhaltenen Salzes oder Solvats.

Die so erhaltenen erfindungsgemäßen Salze können ggf. weiterbearbeitet, z.B. umkristallisiert oder mikronisiert werden, um deren physikalische Eigenschaften weiter auf den Verwendungszweck anzupassen.

Die zur Herstellung der erfindungsgemäßen Salze verwendete {8-Fluor-2-[4-(3-methoxyphenyl)piperazin-1-yl]-3-[2-methoxy-5-(trifluormethyl)phenyl]-3,4-dihydrochinazolin-4-yl}essigsäure ist bekannt und kann zum Beispiel nach dem in der WO 2006/133822 beschriebenen Verfahren hergestellt werden.

Insbesondere erfolgt die Herstellung durch die Verseifung des Esters einer Verbindung der Formel (II) 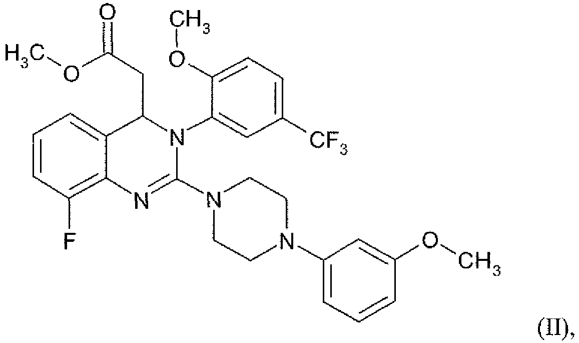 mit einer Base.

Die Verbindung der Formel (II) kann hergestellt werden durch Umsetzung einer Verbindung der Formel (III) 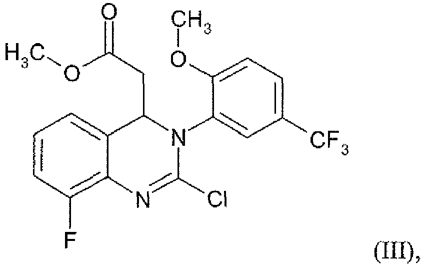 in Gegenwart einer Base mit einer Verbindung der Formel (IV)

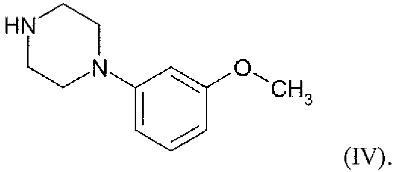

Die Verbindung der Formel (III) kann hergestellt werden durch Umsetzung einer Verbindung der Formel (V) 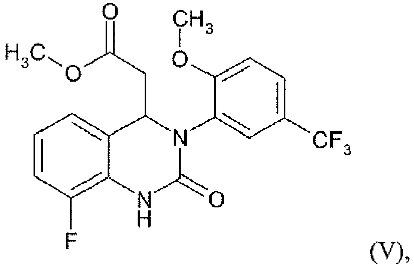 mit Phosphoroxychlorid, Phosphortrichlorid oder Phosphorpentachlorid in Gegenwart einer Base.

Die Verbindung der Formel (V) kann hergestellt werden durch Umsetzung einer Verbindung der Formel (VI) 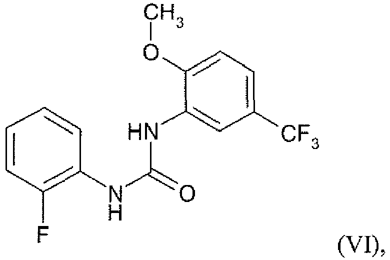 in der ersten Stufe mit Acrylsäuremethylester in Gegenwart eines Palladiumkatalysators und Oleum und in der zweiten Stufe mit einer Base.

Verbindungen der Formeln (IV) und (VI) sind dem Fachmann an sich bekannt oder lassen sich nach üblichen, literaturbekannten Verfahren herstellen.

Die Verseifung des Esters einer Verbindung der Formel (II) zu {8-Fluor-2-[4-(3-methoxyphenyl)piperazin-1-yl]-3-[2-methoxy-5-(trifluormethyl)phenyl]-3,4-dihydrochinazolin-4-yl}essigsäure erfolgt durch Umsetzung einer Verbindung der Formel (II) mit einer Base in einem inerten Lösungsmittel, in einem Temperaturbereich von 18°C bis zum Rückfluss des Lösungsmittels, bevorzugt bei 18 bis 50°C, besonders bevorzugt bei 20 bis 30°C, bei Normaldruck, innerhalb von beispielsweise 0,5 bis 10 Stunden, bevorzugt innerhalb von 1 bis 5 Stunden.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Alkoholate wie Natrium- oder Kaliummethanolat oder Natrium- oder Kaliumethanolat, wobei die Base ggf. in wässriger Lösung vorliegt.

Inerte Lösungsmittel sind beispielsweise Ether wie 1,2-Dimethoxyethan, Methyl-*tert-*butylether (MTBE), Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, oder Wasser, oder Gemische von Lösungsmitteln.

Bevorzugt ist Natriumhydroxid in Wasser und MTBE.

Die Synthese einer Verbindung der Formel (II) aus einer Verbindung der Formel (III) und einer Verbindung der Formel (IV) in Gegenwart einer Base erfolgt in einem inerten Lösungsmittel, in einem Temperaturbereich von 40°C bis zum Rückfluss des Lösungsmittels, bevorzugt bei Rückfluss des Lösungsmittels, bei Normaldruck, innerhalb von beispielsweise 2 bis 48 Stunden, bevorzugt innerhalb von 4 bis 12 Stunden.

Basen sind beispielsweise Aminbasen wie 1,8-Diazabicyclo[5.4.0]undec-7-en (DBU), 1-(3-Methoxyphenyl)piperazin oder Triethylamin, oder andere Basen wie Kalium-tert.-butylat.

Inerte Lösungsmittel sind beispielsweise Chlorbenzol oder Ether wie 1,2-Dimethoxyethan, Dioxan, Glykoldimethylether oder Diethylenglykoldimethylether.

Bevorzugt ist DBU in Dioxan.

Die Umsetzung einer Verbindung der Formel (V) zu einer Verbindung der Formel (III) erfolgt durch Reaktion einer Verbindung der Formel (V) mit Phosphoroxychlorid, Phosphortrichlorid oder Phosphorpentachlorid, bevorzugt ist Phosphoroxychlorid, in Gegenwart einer Base in einem inerten Lösungsmittel, in einem Temperaturbereich von 40°C bis zum Rückfluss des Lösungsmittels, bevorzugt bei Rückfluss des Lösungsmittels, bei Normaldruck, innerhalb von beispielsweise 1 bis 48 Stunden, bevorzugt innerhalb von 2 bis 12 Stunden.

Basen sind beispielsweise Amine wie 1,8-Diazabicyclo[5.4.0]undec-7-en (DBU), Pyridin oder Triethylamin, oder andere Basen wie Kalium-tert.-butylat.
Inerte Lösungsmittel sind beispielsweise Kohlenwasserstoffe wie Benzol, Xylol, Toluol oder Chlorbenzol.

Bevorzugt ist DBU in Chlorbenzol.

Die Umsetzung einer Verbindung der Formel (VI) zu einer Verbindung der Formel (V) erfolgt in der ersten Stufe durch Reaktion einer Verbindung der Formel (VI) mit Acrylsäuremethylester in Gegenwart eines Palladiumkatalysators und Oleum in einem Lösungsmittel in einem Temperaturbereich von 0°C bis 40°C, bevorzugt bei Raumtemperatur und in der zweiten Stufe durch Reaktion mit einer Base in einem inerten Lösungsmittel, in einem Temperaturbereich von 40°C bis zum Rückfluss des Lösungsmittels, bevorzugt bei Rückfluss des Lösungsmittels, bei Normaldruck, innerhalb von beispielsweise 1 bis 48 Stunden, bevorzugt innerhalb von 2 bis 12 Stunden.

Palladiumkatalysatoren in der ersten Stufe sind beispielsweise Palladium(II)acetat, Bis(triphenylphosphin)palladium(II)chlorid, Tetrakis(triphenylphosphin)palladium(0), Bis(tris(o-tolyl)phosphino)palladium-(II)-chlorid oder ein aus Bis(acetonitril)-dichlorpalladium oder Palladium(II)acetat und einem Liganden, beispielsweise Tris(o-tolyl)phosphin, Triphenylphosphin oder Diphenylphosphinoferrocen, hergestellter Palladiumkatalysator.

Lösungsmittel in der ersten Stufe sind beispielsweise organische Säuren wie Essigsäure oder Propionsäure.

Bevorzugt ist Palladium(II)acetat in Essigsäure.

Basen in der zweiten Stufe sind beispielsweise DBU, Triethylamin oder Diisopropylethylamin.

Inerte Lösungsmittel in der zweiten Stufe sind beispielsweise Ether wie 1,2-Dimethoxyethan, Dioxan, Glykoldimethylether oder Diethylenglykoldimethylether, Kohlenwasserstoffe wie Benzol, Xylol oder Toluol, oder andere Lösungsmittel wie Isobutyronitril, Acetonitril, Aceton, Nitrobenzol, Dimethylformamid, Dimethylacetamid, Dimethylsulfoxid oder N-Methylpyrrolidon.

Bevorzugt ist DBU in Aceton.

Die Herstellung der zur Herstellung der erfindungsgemäßen Salze verwendeten {8-Fluor-2-[4-(3-methoxyphenyl)piperazin-1-yl]-3-[2-methoxy-5-(trifluormethyl)phenyl]-3,4-dihydrochinazolin-4-yl}essigsäure ist in dem nachstehenden Syntheseschema 1 exemplarisch näher erläutert. Das Syntheseschema ist hierbei rein beispielhaft und in keiner Weise einschränkend zu verstehen. 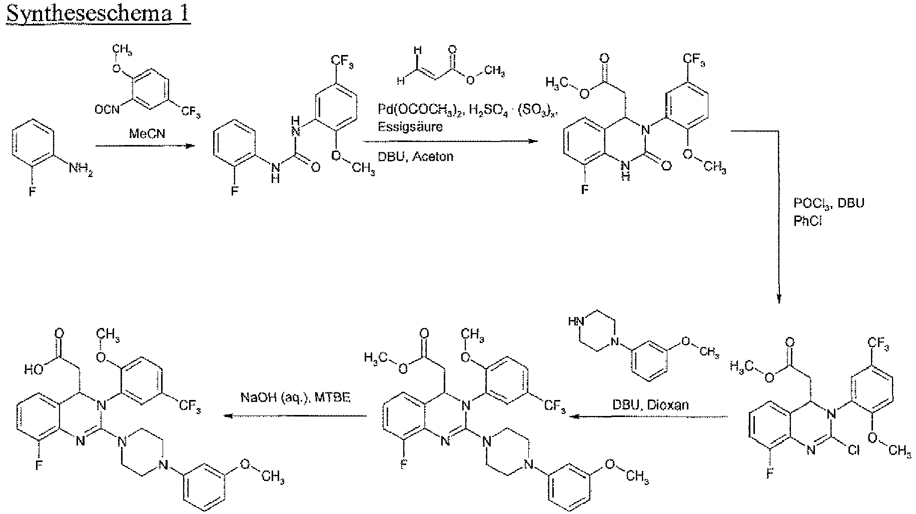 Wie bereits zuvor erwähnt wird die {8-Fluor-2-[4-(3-methoxyphenyl)piperazin-1-yl]-3-[2-methoxy-5-(trifluormethyl)phenyl]-3,4-dihydrochinazolin-4-yl}essigsäure vorzugsweise in Form des S-Enantiomers eingesetzt. Dieses S-Enantiomer kann dabei z.B. wie in dem nachstehenden Syntheseschema 2 dargestellt hergestellt werden. 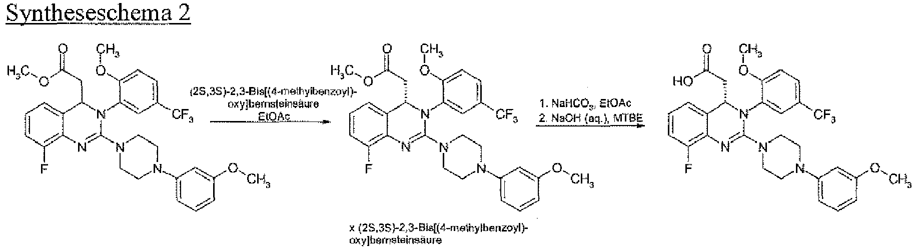 Die erfindungsgemäßen Salze zeigen eine antivirale Wirkung gegenüber Vertretern der Gruppe der Herpes viridae (Herpesviren), vor allen Dingen gegenüber Cytomegalieviren (CMV) insbesondere gegenüber dem humanen Cytomegalievirus (HCMV). Sie sind somit geeignet zur Behandlung und Prophylaxe von Krankheiten, vor allem von Infektionen mit Viren, insbesondere den hierin genannten Viren und den dadurch hervorgerufenen Infektionskrankheiten. Unter einer Virusinfektion wird hierin sowohl eine Infektion mit einem Virus als auch eine durch eine Infektion mit einem Virus hervorgerufene Krankheit verstanden.

Die erfindungsgemäßen Salze können aufgrund ihrer Eigenschaften zur Herstellung von Arzneimitteln verwendet werden, die zur Prophylaxe und/oder Behandlung von Krankheiten, insbesondere Virusinfektionen geeignet sind.

Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalievirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.
5) Behandlung der HCMV-Infektion bei immunsupprimierten Patienten bei Krebs und Krebs-Therapie.
6) Behandlung von HCMV-positiven Krebspatienten mit dem Ziel, HCMVvermittelte Tumorprogression zu verringern (vgl. J. Cinatl , et al., FEMS Microbiology Reviews 2004, 28, 59-77).

Bevorzugt werden die erfindungsgemäßen Salze zur Herstellung von Arzneimitteln verwendet, die zur Prophylaxe und/oder Behandlung von Infektionen mit einem Vertreter der Gruppe der Herpes viridae, besonders einem Cytomegalievirus, insbesondere dem humanen Cytomegalievirus, geeignet sind.

Die erfindungsgemäßen Salze können aufgrund ihrer pharmakologischen Eigenschaften allein und bei Bedarf auch in Kombination mit anderen Wirkstoffen, insbesondere antiviralen Wirkstoffen wie beispielsweise Valganciclovir, Ganciclovir, Valacyclovir, Acyclovir, Foscarnet, Cidofovir und verwandten Derivaten zur Behandlung und/oder Prävention von Virusinfektionen, insbesondere von HCMV-Infektionen, eingesetzt werden.

Weiterer Gegenstand der vorliegenden Erfindung betrifft die erfindungsgemäßen Salze in einem Verfahren zur Behandlung und/oder Prophylaxe von Erkrankungen, vorzugsweise von Virusinfektionen, insbesondere von Infektionen mit dem humanen Cytomegalievirus (HCMV) oder einem anderen Vertreter der Gruppe der Herpes viridae.

Beschrieben wird die Verwendung der erfindungsgemäßen Salze zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Salze zur Herstellung eines Arzneimittels zur Behandlung und/oder Prophylaxe von Erkrankungen, insbesondere der zuvor genannten Erkrankungen.

Beschrieben wird ein Verfahren zur Behandlung und/oder Prophylaxe von Erkrankungen, insbesondere der zuvor genannten Erkrankungen, unter Verwendung einer antiviral wirksamen Menge der erfindungsgemäßen Salze.

Die erfindungsgemäßen Salze können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat beziehungsweise Stent.

Für diese Applikationswege können die erfindungsgemäßen Salze in geeigneten Applikationsformen verabreicht werden.

Für die orale Applikation eignen sich nach dem Stand der Technik funktionierende schnell und/oder modifiziert die erfindungsgemäßen Salze abgebende Applikationsformen, die die erfindungsgemäßen Verbindungen in kristalliner und/oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophilisate, Kapseln (beispielsweise Hart- oder Weichgelatinekapseln), Dragees, Granulate, Pellets, Pulver, Emulsionen, Suspensionen, Aerosole oder Lösungen.
Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subkutan, intrakutan, perkutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wässrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Salze können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglykole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und / oder Geruchskorrigentien.

Weiterer Gegenstand der vorliegenden Erfindung sind Arzneimittel, die mindestens ein erfindungsgemäßes Salz, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen, bezogen auf den reinen Wirkstoff, von etwa 0,001 bis 10 mg/kg, vorzugsweise etwa 0,01 bis 5 mg/kg Körpergewicht zur Erzielung wirksamer Ergebnisse zu verabreichen. Bei oraler Applikation beträgt die Dosierung üblicherweise etwa 0,01 bis 25 mg/kg, vorzugsweise 0,1 bis 10 mg/kg Körpergewicht.

Trotzdem kann es ggf. erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt beziehungsweise Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun nachstehend anhand von Beispielen sowie in Bezug auf die beiliegenden Zeichnungen näher erläutert. Es zeigen:
- Fig. 1: ein Pulver XRD-Diffraktogramm eines 3-Hydrats des Natriumsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure, das gemäß Beispiel 2 hergestellt wurde; und
- Fig. 2: ein Pulver XRD-Diffraktogramm eines 2,5-Hydrats des Calciumsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure, das gemäß Beispiel 3 hergestellt wurde; und
- Fig. 3: ein Pulver XRD-Diffraktogramm eines 3,5-Hydrats des Calciumsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure, das gemäß Beispiel 4 hergestellt wurde.
- Fig. 4: HPLC Chromatogramm für S(+)-{8-Fluor-2-[4-(3-methoxyphenyl)piperazin-1-yl]-3-[2-methoxy-5-(trifluormethyl)phenyl]-3,4-dihydrochinazolin-4-yl}essigsäure Natrium-Salz.
- Fig. 5: Analyse HPLC-Chromatogramm gemäß Fig.4 in Tabellenform.

Die Prozentangaben in den folgenden Tests und Beispielen sind, sofern nicht anders angegeben, Gewichtsprozent, Teile sind Gewichtsteile. Lösungsmittelverhältnisse, Verdünnungsverhältnisse und Konzentrationsangaben von Flüssiglösungen beziehen sich jeweils auf das Volumen.

### Abkürzungsverzeichnis:

- ACN: Acetonitril
- API: active pharmaceutical ingredient
- API-ES-pos.: Atmospheric pressure ionization, electrospray, positive (bei MS)
- API-ES-neg.: Atmospheric pressure ionization, electrospray, negative (bei MS)
- ca.: circa
- CI, NH₃: chemische Ionisierung (mit Ammoniak)
- DBU: 1,8-Diazabicyclo[5.4.0]undec-7-en
- DMAP: 4-(Dimethylamino)pyridin
- DMSO: Dimethylsulfoxid
- ESTD: externe Standardisierung
- h: Stunde(n)
- HPLC: Hochdruckflüssigchromatographie (high pressure liquid chromatography)
- konz.: konzentriert(e)
- min.: Minuten
- MS: Massenspektroskopie
- MTBE: Methyl-*tert*-butylether
- NMR: Kernresonanzspektroskopie (nuclear magnetic resonance spectroscopy)
- R_{T}: Retentionszeit (bei HPLC)
- VTS: Vakuumtrockenschrank

### Allgemeine Methoden HPLC:

**Methode 1** (HPLC): Instrument: HP 1050 mit variabler Wellenlängen-Detektion; Säule: Phenomenex-Prodigy ODS (3) 100A, 150 mm x 3 mm, 3 µm; Eluent A: (1,0 g KH₂PO₄ + 1,0 mL H₃PO₄) / 1 Wasser, Eluent B: Acetonitril; Gradient: 0 min 10% B, 25 min 80% B, 35 min 80% B; Fluss: 0,5 ml/min; Temp.: 45°C; UV-Detektion: 210 nm.

**Methode 2** (HPLC): Instrument: HP 1050 mit variabler Wellenlängen-Detektion; Säule: Chiral AD-H, 250 mm x 4,6 mm, 5 µm; Eluent A: *n*-Heptan + 0,2 % Diethylamin, Eluent B: Isopropanol + 0,2 % Diethylamin; Gradient: 0 min 12,5 % B, 30 min 12,5 % B; Fluss: 1 ml/min; Temp.: 25°C; UV-Detektion: 250 um.

**Methode 3** (HPLC): Instrument: HP 1050 mit variabler Wellenlängen-Detektion; Säule: Chiral AD-H, 250 mm x 4,6 mm, 5 µm; Eluent A: *n*-Heptan + 0,2 % Diethylamin, Eluent B: Isopropanol + 0,2 % Diethylamin; Gradient: 0 min 25 % B, 15 min 25 % B; Fluss: 1 ml/min; Temp.: 30°C; UV-Detektion: 250 nm.

### Beispiele

### A) Herstellung von {8-Fluor-2-[4-(3-methoxyphenyl)piperazin-1-yl]-3-[2-methoxy-5-(trifluormethyl)phenyl]-3,4-dihydrochinazolin-4-yl}essigsäure

### Beispiel 1A

*N-*(2-Fluorphenyl)-*N'*-[2-methoxy-5-(trifluormethyl)phenyl]harnstoff 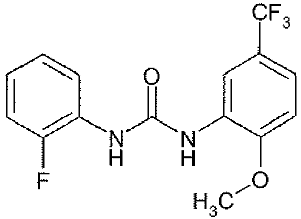 2-Methoxy-5-trifluormethylphenylisocyanat (78 kg) wird bei ca. 35°C aufgeschmolzen und in Acetonitril (insgesamt ca. 270 1) gelöst, dann wird 2-Fluoranilin (39,9 kg) zugegeben und mit Acetonitril (ca. 25 1) nachgespült. Die resultierende klare Lösung wird 4 h unter Rückfluss gerührt und dann auf ca. 75°C abgekühlt. Bei dieser Temperatur wird die Lösung mit Impfkristallen des gewünschten Endprodukts (200 g) angeimpft, für weitere 15 min. gerührt und dann über 3 h auf 0°C abgekühlt. Das erhaltene kristalline Produkt wird durch Zentrifugieren isoliert mit kaltem Acetonitril (zweimal ca. 13 1) gewaschen und bei 45°C im VTS mit Schleppstickstoff getrocknet (ca. 3,5 h). So werden insgesamt 101,5 kg N*-(2-Fluorphenyl)-*N'*-[2-methoxy-5-(trifluormethyl)phenyl]harnstoff* als Feststoff erhalten, entsprechend 85,9 % der Theorie.

¹H NMR (300 MHz, d₆-DMSO): δ = 8,93 (s, 1H), 8,84 (s, 1H), 8,52 (d, ³*J* = 2,3, 2H), 7,55 (d, ²*J* = 7,7, 1H), 7,38-7,26 (m, 3H), 7,22 (d, ²*J* = 8,5, 1H), 4,00 (s, 3H) ppm;
MS (API-ES-pos.): m/z = 409 [(M+H)⁺, 100 %];
HPLC (Methode 1): R_{T} = 22,4 und 30,6 min.

### Beispiel 2A

Methyl-(2Z)-3-[3-fluor-2-({[2-methoxy-5-(trifluormethyl)phenyl]carbamoyl}amino)-phenyl]acrylat 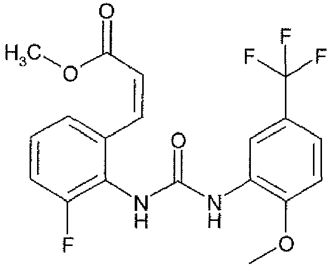 In einem ersten Reaktor werden *N*-(2-Fluorphenyl)-*N*'-[2-methoxy-5-(trifluormethyl)phenyl]harnstoff (51 kg) unter einer Stickstoff-Atmosphäre in Essigsäure (ca. 4301) gelöst. Zu der entstandenen Lösung wird Methylacrylat (20,1 kg) zugegeben und die entstandene Suspension wird bis zur weiteren Verwendung gerührt. In einem zweiten Reaktor wird Essigsäure (950 l) vorgelegt, Oleum (57 kg) vorsichtig zugegeben und Palladium(II)acetat (7 kg) in der erhaltenen Mischung gelöst. Die in dem ersten Reaktor gebildete Suspension wird nun über ca. 2 h zu der in dem zweiten Reaktor enthaltenen Mischung zugegeben, wobei das Reaktionsgemisch mit einem Gemisch aus 96% Stickstoff und 4% Sauerstoff überströmt wird und die erhaltene Reaktionsmischung wird für ca. 18 h bei Raumtemperatur gerührt. Anschließend wird ein Teil der Essigsäure (ca. 900 l) abdestilliert, zu der verbleibenden Reaktionsmischung wird über ca. 1 h Wasser (ca. 5001) zugegeben und die erhaltene Suspension wird für 1 h gerührt. Der erhaltene Feststoff wird abfiltriert, einmal mit einem Gemisch aus Essigsäure und Wasser (1 : 1) und zweimal mit Wasser gewaschen und anschließend bei ca. 30 mbar und 50°C getrocknet. So werden insgesamt 44,8 kg *Methyl*-(*2Z*)-*3*-[*3*-*fluor-2*-*({[2*-*methoxy*-*5*-*(trifluormethyl*)*phenyl]carbamoyl}-amino)phenyl]acrylat* als Feststoff erhalten, entsprechend 65,0 % der Theorie.

¹H NMR (300 MHz, d₆-DMSO): δ = 9,16 (s, 1H), 8,84 (s, 1H), 8,45 (d, 1,7Hz, 1H), 7,73 (m, 2H), 7,33 (m, 3H), 7,22 (d, 8,6Hz, 1H), 6,70 (d, 16Hz, 1H), 3,99 (s, 3H), 3,71 (s, 3H) ppm;
MS (API-ES-pos.): m/z = 429,9 [(M+NH₄)⁺]; 412,9 [(M+H)⁺]
HPLC:R_{T}= 46,4 min.

Instrument: HP 1100 mit variabler Wellenlängen-Detektion; Säule: Phenomenex-Prodigy ODS (3) 100A, 150 mm x 3 mm, 3 µm; Eluent A: (1,36 g KH₂PO₄ + 0,7 ml H₃PO₄) / l Wasser, Eluent B: Acetonitril; Gradient: 0 min 20% B, 40 min 45% B, 50 min 80% B, 65 min 80% B; Fluss: 0,5 ml/min; Temp.: 55°C; UV-Detektion: 210 nm.

### Beispiel 3A

{8-Fluor-3-[2-methoxy-5-(trifluormethyl)phenyl]-2-oxo-1,2,3,4-tetrahydrochinazolin-4-yl}essigsäuremethylester 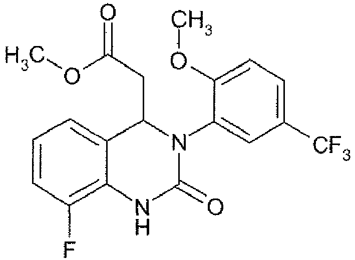 Die Verbindung von Beispiel 2A (75 kg) wird in Aceton (1600 l) suspendiert und DBU (5,7 kg) wird zugegeben. Die resultierende Suspension wird zum Rückfluss erwärmt und 4 h unter Rückfluss gerührt. Die erhaltene Lösung wird auf eine Manteltemperatur von 55°C abgekühlt und über Kieselgur filtriert. Ein Teil des Lösungsmittels wird durch Destillation aus dem Reaktionsgemisch entfernt (ca. 1125 l) und der verbleibende Rückstand wird für 2 h auf 0°C abgekühlt. Der entstandene Feststoff wird durch Zentrifugieren abgetrennt und zweimal mit kaltem Aceton (ca. 15 l) gewaschen und über Nacht bei 45°C unter reduziertem Druck mit Schleppstickstoff bis zur Massenkonstanz getrocknet. So werden insgesamt 58,3 kg *{8-Fluor-3-[2-methoxy-5-(trifluormethy)phenyl]-2-oxo-1*,*2*,*3*,*4-tetrahydrochinazolin-4*-*yl}essigsäure*-*methylester* als Feststoff erhalten, entsprechend 84,1 % der Theorie.
HPLC (Methode 1): R_{T} = 19,4 min.

### Beispiel 4A

(2*S*,3*S*)-2,3-Bis[(4-methylbenzoyl)oxy]bernsteinsäure-{(4*S*)-8-Fluor-2-[4-(3-methoxyphenyl)-piperazin-1-yl]-3-[2-methoxy-5-(trifluormethyl)phenyl]-3,4-dihydrochinazolin-4-yl}-essigsäuremethylester (1:1-Salz) Chlorierung/Aminierung/Kristallisation 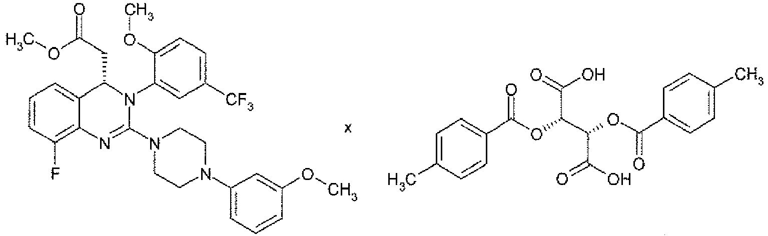 Eine Lösung von {8-Fluor-3-[2-methoxy-5-(trifluormethyl)phenyl]-2-oxo-1,2,3,4-tetrahydrochinazolin-4-yl}essigsäuremethylester (Beispiel 3A, 129,2 kg) in Chlorbenzol (800 1) wird auf Rückfluss erhitzt und azeotrop getrocknet. Phosphoroxychlorid (144 kg) wird zugegeben, und die Reaktionsmischung wird 3 h unter Rückfluss gerührt. Anschlieend wird DBU (95 kg) und Chlorbenzol (45 l) zugegeben und für weitere 9 h unter Rückfluss gerührt. Die Reaktionsmischung wird auf Raumtemperatur abgekühlt, durch Zugabe in Wasser hydrolysiert, mit Chlorbenzol (80 l) verdünnt und mit wässriger Ammoniaklösung (25%) neutralisiert. Die Phasen werden getrennt und die organische Phase mit Wasser gewaschen und das Lösungsmittel abdestilliert. Der verbleibende Rückstand wird in Dioxan (170 l) gelöst. 3-Methoxyphenylpiperazin (66 kg), DBU (52 kg) und weitere 90 l Dioxan werden zugegeben und die Reaktionsmischung wird 4 h unter Rückfluss erwärmt. Die Reaktionsmischung wird auf Raumtemperatur abgekühlt, mit Essigester (1300 l) versetzt, 1x mit Wasser, 3 x mit 0.2 N HCl, und 1x mit wässriger NaCl-Lösung gewaschen und das Lösungsmittel wird abdestilliert. Der erhaltene Rückstand wird in Essigester (800 l) gelöst und in eine Lösung aus (2*S*,3*S*)-2,3-Bis[(4-methylbenzoyl)oxy]bernsteinsäure (121 kg) in Essigester (600 l) gegeben. Das resultierende Gemisch wird ca. 60 min. bei Raumtemperatur gerührt, dann wird mit (2*S*,3*S*)-2,3-Bis[(4-methylbenzoyl)oxy]-bernsteinsäure-{(4*S*)-8-Fluor-2-[4-(3-methoxyphenyl)piperazin-1-yl]-3-[2-methoxy-5-(trifluormethyl)-phenyl]-3,4-dihydrochinazolin-4-yl}essigsäuremethylester angeimpft und 3 Tage bei Raumtemperatur gerührt. Anschließend wird auf 0 - 5°C gekühlt und weitere 3 h gerührt. Die Suspension wird abgesaugt und portionsweise mit Ethylacetat nachgewaschen. So werden insgesamt etwa 141 kg (berechnet als trocken) des Salzes als Feststoff erhalten, entsprechend etwa 46,2 % der Theorie über drei Stufen (Chlorierung, Aminierung und Kristallisation) bezogen auf das Racemat.

¹H NMR (300 MHz, d₆-DMSO): δ = 7,90 (d, ²*J* = 7,8, 4H), 7,56 (d, ²*J* = 8,3, 1H), 7,40 (d, ²*J* = 7,8, 4H), 7,28-7,05 (m, 4H), 6,91-6,86 (m, 2H), 6,45 (d, *²J* = 8,3, 1H), 6,39-6,36 (m, 2H), 5,82 (s, 2H), 4,94 (m, 1H), 4,03 (q, ²*J* = 7,1, 2H), 3,83 (brs, 3H), 3,69 (s, 3H), 3,64 (s, 3H), 3,47-3,36 (m, 8H und Wasser, 2H), 2,98-2,81 (m, 5H), 2,58-2,52 (m, 1H), 2,41 (s, 6H), 1,99 (s, 3H), 1,18 (t, ²*J* = 7,2, 3H) ppm;
HPLC (Methode 1): R_{T} = 16,6 und 18,5 min.

### Beispiel 5A

(2*S*,3*S*)-2,3-Bis[(4-methylbenzoyl)oxy]bernsteinsäure-{(4*S*)-8-Fluor-2-[4-{3-methoxyphenyl}piperazin-1-yl]-3-[2-methoxy-5-{trifluormethyl}phenyl]-3,4-dihydrochinazolin-4-yl}essigsäuremethylester (1:1-Salz) / Umkristallisation (2S,3S)-2,3-Bis[(4-methylbenzoyl)oxy]bernsteinsäure - (S) {(4*S*)-8-Fluor-2-[4-(3-methoxyphenyl)piperazin-1-yl]-3-[2-methoxy-5-(trifluormethyl)phenyl]-3,4-dihydrochinazolin-4-y1}essigsäuremethylester (1:1-Salz) (141 kg, berechnet als trocken) wird in Ethylacetat (1400 1) suspendiert und durch Erhitzen auf Rückfluss (77°C) gelöst. Die Lösung wird filtriert und langsam auf Raumtemperatur gekühlt. Dabei erfolgt eine spontane Kristallisation. Die Suspension wird 16 h bei RT gerührt, anschließend auf 0-5°C gekühlt und weitere 3 h gerührt. Die Suspension wird abgesaugt und mit kaltem Ethylacetat nachgewaschen. Die Kristalle werden 16 h im Vakuum bei etwa 40 °C getrocknet. So werden insgesamt 131,2 kg des Salzes als Feststoff erhalten, entsprechend 93,0 % der Theorie.
HPLC (Methode 1): R_{T} = 16,9 und 18,8 min.;
HPLC (Methode 3): 99,9 % e.e.

### Beispiel 6A

(S)-{8-Fluor-2-[4-(3-methoxyphenyl)piperazin-1-yl]-3-(2-methoxy-5-trifluormethylphenyl)-3,4-dihydrochinazolin-4-yl} essigsäure 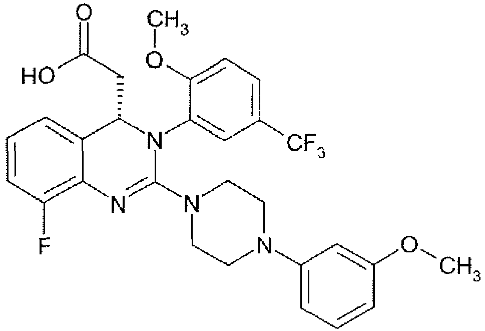 Eine Mischung aus (2*S*,3*S*)-2,3-Bis[(4-methylbenzoyl)oxy]bernsteinsäure-{(4*S*)-8-Fluor-2-[4-(3-methoxyphenyl)piperazin-1-yl]-3-[2-methoxy-5-(trifluormethyl)phenyl]-3,4-dihydrochinazolin-4-yl}essigsäuremethylester (1:1-Salz) (30,8 kg), Natriumhydrogencarbonat (16.4 kg) und Wasser (315 l) wird mit MTBE (160 l) verrührt. Die Phasen werden getrennt und die organische Phase mit 35 l einer etwa siebenprozentigen wässrigen Natriumhydrogencarbonatlösung behandelt. Die Phasen werden getrennt und die organische Phase wird mit 125 1 einer etwa vierprozentigen wässrigen Natriumhydroxidlösung versetzt. Die Reaktionsmischung wird zum Rückfluss erhitzt, das Lösungsmittel schonend bis zum Versiegen destilliert und der Reaktorinhalt anschließend weitere 5 h bei 55 - 60 °C gerührt. Die Reaktionsmischung wird dann bei etwa 22 °C mit MTBE (160 l) und Wasser (65 l) versetzt und verrührt. Die Phasen werden getrennt und die organische Phase mit einer etwa sechsprozentigen wässrigen Natriumchloridlösung (30 1) extrahiert. Die vereinigten wässrigen Phasen werden mit Wasser (25 l) und MTBE (160 l) verrührt und der pH-Wert mit etwa IN Salzsäure auf etwa 6,5 gestellt. Die organische Phase wird abgetrennt, das Lösungsmittel schonend bis zum Versiegen destilliert und der Rückstand in Aceton (ca. 75 l) gelöst. Es wird ein Lösungsmittelaustausch zu Aceton (6 Destillationsvorgänge mit je ca. 130 l) durchgeführt. Das Zielprodukt wird anschließend durch Zugeben in Wasser ausgefällt, durch Zentrifugieren isoliert und im Vakuumtrockner getrocknet. So werden insgesamt 16,5 kg *(S)-{8-Fluor-2-[4-(3-methoxyphenyl)piperazin-1-yl]-3-(2-Methoxy-5-trifluormethylphenyl)-3,4-dihydrochinazolin-4-yl}essigsäure* als amorpher Feststoff erhalten, entsprechend 96,4 % der Theorie.

¹H NMR (300 MHz, d₆-DMSO): δ = 7,53 (d, ²*J* = 8,4, 1H), 7,41 (brs, 1H), 7,22 (d, *²J* = 8,5, 1H), 7,09-7,01 (m, 2H), 6,86 (m, 2H), 6,45 (dd, ²*J =* 8,2, ³*J* = 1,8, 1H), 6,39-6,34 (m, 2H), 4,87 (t, ²*J* = 7,3, 1H), 3,79 (brs, 3H), 3,68 (s, 3H), 3,50-3,38 (m, 4H), 2,96-2,75 (m, 5H), 2,45-2,40 (m, 1H) ppm;
MS (API-ES-neg.): m/z = 571 [(M-H), 100 %];
HPLC (Methode 1): R_{T} = 15,1 min;
HPLC (Methode 2): 99,8 % e.e.; Pd (ICP): <1 ppm.

### B) Ausführungsbeispiele

### Kristallisationsversuche

Kristallisationsversuche zur Auffindung eines geeigneten kristallinen Salzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure wurden sowohl für Säuresalze als auch für Basensalze durchgeführt. Die Kristallisationsversuche erfolgten ausgehend von *(S)*-{8-Fluor-2-[4-(3-methoxyphenyl)piperazin-1-yl]-3-[2-methoxy-5-(trifluormethyl)phenyl]-3,4-dihydrochinazolin-4-yl}essigsäure und der jeweiligen Säure bzw. Base entweder durch Aufschlämmen im jeweils angegebenen Lösungsmittel für eine Woche bei 20°C oder durch eine Kristallisation durch Abkühlen/Abdampfen ausgehend von einer Lösung, die für 4 Stunden bei 50°C gehalten wurde, gefolgt von einem langsamen Abkühlen auf 20°C mit einer Geschwindigkeit von 3°C/Stunde.

Die Ergebnisse für die Kristallisationsversuche mit Säuren bzw. Basen sind nachstehend in Tabelle 1 und Tabelle 2 angegeben, wobei die Abkürzung API für *(S)*-{8-Fluor-2-[4-(3-methoxyphenyl)piperazin-1-yl]-3-[2-methoxy-5-(trifluormethyl)phenyl]-3,4-dihydrochinazolin-4-yl}essigsäure steht.

"API" ist das Akronym für "active pharmaceutical ingredient" (aus dem Englischen für aktiver Arzneiwirkstoff).

**Tabelle 1**

| Kristallisationsversuche unter Verwendung saurer Gegenionen | | | | |
|---|---|---|---|---|
| **Gegenionen** | **Verhältnis API: Gegenionen** | **Verfahren** | **Lösungsmittel** | **Ergebnis (XRPD)** |
| HCL | 1:2 | Abkühlen | Aceton, Acetonitril, Methanol, THF | amorph |
| | | Aufschlämmen | Wasser, Acetonitril, Methanol und Ethanol | |
| Citronensäure | 1:1 | Abkühlen | Aceton, Acetonitril, Methanol, THF | amorph |
| | | Aufschlämmen | Wasser, Acetonitril Methanol und Ethanol | |
| Phosphorsäure | 1:1 | Abkühlen | Aceton, Acetonitril, Methanol, THF | amorph |
| | | Aufschlämmen | Wasser, Acetonitril, Methanol und Ethanol | |
| Gluconsäure | 1:1 | Abkühlen | Aceton, Acetonitril, Methanol, THF | amorph |
| | | Aufschlämmen | Wasser, Acetonitril Methanol und Ethanol | |
| Milchsäure | 1:1 | Abkühlen | Aceton, Acetonitril, Methanol, THF | amorph |
| | | Aufschlämmen | Wasser, Acetonitril Methanol und Ethanol | |
| Maleinsäure | 1:1 | Abkühlen | Aceton, Acetonitril, Methanol, THF | amorph |
| | | Aufschlämmen | Wasser, Acetonitril Methanol und Ethanol | |
| Bernsteinsäure | 1:1 | Abkühlen | Aceton, Acetonitril, Methanol, THF | amorph |
| | | Aufschlämmen | Wasser, Acetonitril Methanol und Ethanol | |
| Schwefelsäure | 1:1 | Abkühlen | Aceton, Acetonitril, Methanol, THF | amorph |
| | | Aufschlämmen | Wasser, Acetonitril Methanol und Ethanol | |
| Weinsäure | 1:1 | Abkühlen | Aceton, Acetonitril, Methanol, THF | amorph |
| | | Aufschlämmen | Wasser, Acetonitril Methanol und Ethanol | |
| Benzoesäure | 1:1 | Abkühlen | Aceton, Acetonitril, Methanol, THF | amorph |
| | | Aufschlämmen | Wasser, Acetonitril Methanol und Ethanol | |
| Fumarsäure | 1:1 | Abkühlen | Aceton, Acetonitril, Methanol, THF | amorph |
| | | Aufschlämmen | Wasser, Acetonitril Methanol und Ethanol | |
| Maleinsäure | 1:1 | Abkühlen | Aceton, Acetonitril, Methanol, THF | amorph |
| | | Aufschlämmen | Wasser, Acetonitril Methanol und Ethanol | |
| Methansulfonsäure | 1:1 | Abkühlen | Aceton, Acetonitril, Methanol, THF | amorph |
| | | Aufschlämmen | Wasser, Acetonitril Methanol und Ethanol | |

**Tabelle 2**

| Kristallisationsversuche unter Verwendung basischer Gegenionen | | | |
|---|---|---|---|
| **Gegenionen** | **Verfahren** | **Lösungsmittel** | **Ergebnis** |
| NaOH | Abkühlen | Aceton+Isopropylether 1:1, Acetonitril+Isopropylether 1:1, | kristalline: Methanol, Ethanol amorph: alle |
| | Aufschlämmen | Methanol+Isopropylether 1:1, THF und Isopropylether 1:1 Wasser, Acetonitril, Methanol und Ethanol | anderen |
| Erbumin | Abkühlen | Aceton+Isopropylether 1:1, Acetonitril+Isopropylether 1:1, | amorph |
| | Aufschlämmen | Methanol+Isopropylether 1:1, | |
| | THF | THF und Isopropylether 1:1 Wasser, Acetonitril, Methanol und Ethanol | |
| 2-Amino-2-methylpropanol | Abkühlen | Aceton+Isopropylether Acetonitril+Isopropylether 1:1, | amorph |
| | Aufschlämmen | Methanol+Isopropylether 1:1, THF und Isopropylether 1:1 Wasser, Acetonitril, Methanol und Ethanol | |
| 2-Amino-2-methyl-1,3-propandiol | Abkühlen | Aceton+Isopropylether 1:1, Acetonitril+Isopropylether 1:1, | kristallin: THF, Acetonitril amorph: alle anderen |
| | Aufschlämmen | Methanol+Isopropylether 1:1, THF und Isopropylether 1:1 Wasser, Acetonitril, Methanol und Ethanol | |
| Tromethamin | Abkühlen | Aceton+Isopropylether 1:1, Acetonitril+Isopropylether 1:1, | amorph |
| | Aufschlämmen | Methanol+Isopropylether 1:1, THF und Isopropylether 1:1 Wasser, Acetonitril, Methanol und Ethanol | |
| Dimethylaminoethanol | Abkühlen | Aceton+Isopropylether 1:1, Acetonitril+Isopropylether 1:1, | amorph |
| | Aufschlämmen | Methanol+Isopropylether 1:1, THF und Isopropylether 1:1 Wasser, Acetonitril, Methanol und Ethanol | |
| Lysin | Abkühlen | Aceton+Isopropylether 1:1, Acetonitril+Isopropylether 1:1, | amorph |
| | Aufschlämmen | Methanol+Isopropylether 1:1, THF und Isopropylether 1:1 Wasser, Acetonitril, Methanol und Ethanol | |
| N-(2-Hydroxyethyl)pyrrolidin | Abkühlen | Aceton+Isopropylether 1:1, Acetonitril+Isopropylether 1:1, | amorph |
| aufschlämmen | Aufschlämmen | Methanol+Isopropylether 1:1, THF und Isopropylether 1:1 Wasser, Acetonitril, Methanol und Ethanol | |

Was bei diesen Experimenten auffällig ist, ist dass es sich allgemein als äußerst schwierig erwiesen hat, kristalline Salze von *(S)*-{8-Fluor-2-[4-(3-methoxyphenyl)piperazin-1-yl]-3-[2-methoxy-5-(trifluormethyl)phenyl]-3,4-dihydrochinazolin-4-yl}essigsäure herzustellen, insbesondere ist auffällig, dass die Kristallisation unter Verwendung saurer Gegenionen vollkommen fehlgeschlagen ist.

Das 2-Amino-2-methyl-1,3-propandiolsalz von *(S)*-{8-Fluor-2-[4-(3-methoxyphenyl) piperazin-1-yl]-3-[2-methoxy-5-(trifluormethyl)phenyl]-3,4-dihydrochinazolin-4-yl}essigsäure ist aufgrund dessen, dass dieses nur in organischen Lösungsmitteln wie Tetrahydrofuran und Acetonitril kristallisiert, was zu unerwünschten Verunreinigungen führen kann, für die pharmazeutische Weiterentwicklung eher ungeeignet.

Im Hinblick auf die mit Natriumhydroxid erhaltenen positiven Ergebnisse wurden weitere Kristallisationsversuche unter Verwendung von Alkalimetall- bzw. Erdalkalimetallhydroxiden unter analogen Kristallisationsbedingungen wie für das Natriumsalz durchgeführt. Es hat sich hierbei gezeigt, dass lediglich unter Verwendung von Calciumhydroxid weitere kristalline Salze erhalten werden konnten.

### Beispiel 1

Mononatriumsalz von *(S)*-{8-Fluor-2-[4-(3-methoxyphenyl)piperazin-1-yl]-3-[2-methoxy-5-(trifluormethyl)phenyl]-3,4-dihydrochinazolin-4-yl}essigsäure:
331,1 g *(S)*-{8-Fluor-2-[4-(3-methoxyphenyl)piperazin-1-yl]-3-[2-methoxy-5-(trifluormethyl)phenyl]-3,4-dihydrochinazolin-4-yl}essigsäure (Beispiel 6A) werden in einem 2000 ml Dreihalskolben in 1300 ml einer Mischung aus Ethanol und Diisopropylether (1:1) gelöst. Zu der Lösung werden 21,9 g (546,84 mmol) NaOH als Feststoff zugegeben. Die Mischung wird über 25 min. auf eine Innentemperatur von 50°C erwärmt, wobei eine klare orangegefärbte Lösung erhalten wird. Die so erhaltene Lösung wird 3 h bei dieser Temperatur gerührt, wobei sich nach 1 h bereits eine dünne Suspension bildet. Die Reaktionsmischung wird anschließend über 10 h mit einer Abkühlrate von 3°C/h auf eine Innentemperatur von 20°C abgekühlt und weitere 5 h bei dieser Temperatur gerührt. Unter Vakuum wird das Gesamtvolumen der Reaktionsmischung auf ca. 750 ml reduziert und die erhaltene Suspension bei 20°C für 2 h gerührt. Anschließend werden zu der erhaltenen Reaktionsmischung 250 ml Diisopropylether über 10 min. zugegeben und die erhaltene Mischung wird für weitere 2 h gerührt. Das erhaltene kristalline Produkt wird abgesaugt, 2x mit je 250 ml Diisopropylether gewaschen und bei 20°C und 160 mbar 20 h im Vakuumtrockenschrank getrocknet. Der so erhaltene kristalline Feststoff wird anschließend für 10 min. bei 90°C in einem IR-Trockner und für weitere 16 h bei 60°C erneut im Vakuumtrockenschrank nachgetrocknet. So werden insgesamt 274,4 g (86% der Theorie) des gewünschten kristallinen Natriumsalzes erhalten.

### Beispiel 2

Herstellung des 3-Hydrats des Mononatriumsalzes von *(S)*-{8-Fluor-2-[4-(3-methoxyphenyl)piperazin-1-yl]-3-[2-methoxy-5-(trifluormethyl)phenyl]-3,4-dihydrochinazolin-4-yl}essigsäure:
Ca. 300 mg des Natriumsalzes von Beispiel 1 werden in 1 ml Ethanol (enthält 4 % Wasser) suspendiert und 25°C für eine Woche geschüttelt. Der erhaltene Feststoff wird abfiltriert und der Rückstand bei Raumtemperatur unter Umgebungsfeuchte getrocknet. Der erhaltene Rückstand entspricht der Titelverbindung als Trihydrat.

Der erhaltene Rückstand wurde röntgendiffraktometrisch untersucht. Das dabei erhaltene Diffraktogramm ist in Fig. 1 dargestellt.

Das Röntgendiffraktogramm wurde mit einem XRD-Transmissions/Reflexions-Diffraktometer X'Pert PRO (PANalytical) bei Raumtemperatur aufgenommen (Strahlung: Kupfer, Kα1, Wellenlänge: 1,5406 Å). Es gab keine Probenvorbereitung.

Die Peaklisten für das Salz von Beispiel 2 sowie die Salze der Beispiele 3 und 4 sind nachstehend in Tabelle 3 wiedergegeben.

**Tabelle 3**

| Peaklisten der Pulverdiffraktogramme der Salze von Beispiel 2, 3 und 4 | | |
|---|---|---|
| **2 Theta** | | |
| **Beispiel 2** | **Beispiel 3** | **Beispiel 4** |
| 6,2 | 4,6 | 6,0 |
| 9,4 | 6,1 | 6,2 |
| 11,3 | 6,3 | 7,6 |
| 12,4 | 7,6 | 9,0 |
| 12,9 | 8,8 | 9,4 |
| 15,6 | 9,2 | 9,6 |
| 16,4 | 10,6 | 10,7 |
| 16,8 | 11,0 | 11,2 |
| 17,9 | 11,2 | 11,6 |
| 18,6 | 11,6 | 12,1 |
| 18,9 | 12,2 | 12,4 |
| 19,9 | 12,3 | 13,1 |
| 20,9 | 12,6 | 15,1 |
| 21,5 | 12,8 | 15,4 |
| 21,8 | 13,0 | 16,0 |
| 22,4 | 13,3 | 16,1 |
| 22,7 | 13,9 | 16,3 |
| 23,5 | 14,5 | 16,6 |
| 24,9 | 15,3 | 16,9 |
| 25,2 | 15,5 | 17,4 |
| 25,9 | 16,2 | 17,7 |
| 26,4 | 16,3 | 18,2 |
| 26,7 | 16,5 | 18,3 |
| 27,2 | 16,7 | 18,9 |
| 27,4 | 17,0 | 19,2 |
| 28,1 | 17,5 | 19,5 |
| 28,5 | 17,7 | 19,8 |
| 29,5 | 18,1 | 20,0 |
| 30,1 | 18,5 | 20,5 |
| 30,8 | 18,7 | 20,7 |
| 31,2 | 19,4 | 21,3 |
| 32,1 | 19,7 | 21,8 |
| 32,5 | 20,5 | 22,0 |
| 32,8 | 20,8 | 22,4 |
| 33,3 | 21,3 | 22,7 |
| 34,9 | 21,5 | 23,8 |
| 35,6 | 21,7 | 24,2 |
| 36,2 | 22,2 | 24,5 |
| 36,8 | 22,3 | 25,4 |
| 36,8 | 23,0 | 26,2 |
| 37,9 | 23,3 | 26,3 |
| | 23,7 | 26,5 |
| | 24,2 | 27,0 |
| | 24,8 | 27,5 |
| | 25,4 | 28,3 |
| | 25,9 | 29,4 |
| | 26,3 | 31,5 |
| | 26,5 | 34,1 |
| | 26,9 | 35,9 |
| | 27,3 | 37,0 |
| | 27,9 | 37,3 |
| | 28,2 | |
| | 28,7 | |
| | 29,0 | |
| | 29,3 | |
| | 30,5 | |
| | 31,9 | |
| | 32,4 | |
| | 33,1 | |
| | 34,1 | |
| | 34,1 | |
| | 36,0 | |
| | 37,4 | |

### Beispiel 3

Herstellung des 2,5 Hydrats des Calciumsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure:
10 g *(S)*-{8-Fluor-2-[4-(3-methoxyphenyl)piperazin-1-yl]-3-[2-methoxy-5-(trifluormethyl) phenyl]-3,4-dihydrochinazolin-4-yl}essigsäure (Beispiel 6A) werden in einem 50 ml Dreihalskolben in 45 ml Ethanol gelöst und zu der entstandenen Lösung werden 1,294 g Ca(OH₂) als Feststoff in Form eines Pulvers zugegeben. Die erhaltene Suspension wird über 25 min. auf 50°C erwärmt und dann 3 h bei dieser Temperatur nachgerührt. Zu der auf diese Weise erhaltenen Suspension werden 62,6 g Wasser zugegeben und die dabei erhaltene Lösung wird auf 0°C abgekühlt. Zu der so erhaltenen Lösung werden vorher erzeugte Impfkristalle zugegeben und die ein teilweise öliges Produkt aufweisende Suspension wird auf Raumtemperatur erwärmt und 72 h bei Raumtemperatur stehengelassen. Die erhaltene Suspension wird erneut auf 0°C abgekühlt und 2 h bei dieser Temperatur nachgerührt. Das erhaltene kristalline Produkt wird abfiltriert und 2x mit je 15 ml einer Mischung aus Ethanol und Wasser 1:1 gewaschen und dann bei 50°C und 160 mbar im Vakuumtrockenschrank getrocknet. Insgesamt werden 7,4 g (68,5% der Theorie) des Salzes als kristalliner Feststoff erhalten.

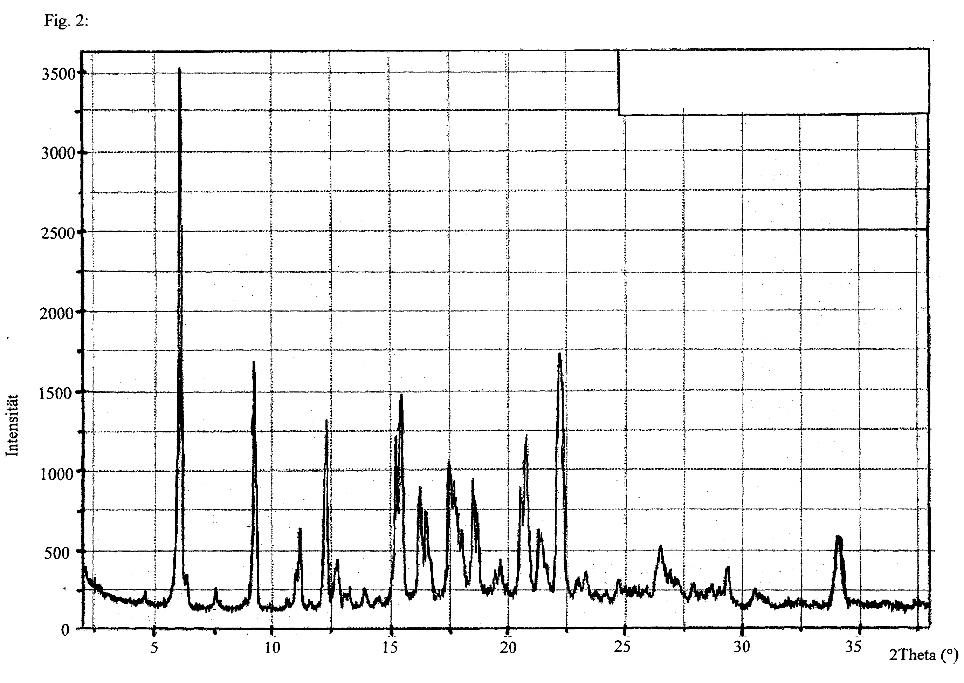

Unter Verwendung des im Beispiel 3 erhaltenen kristallinen Feststoffs wurde unter den gleichen Bedingungen wie in Beispiel 2 genannt ein Pulver-XRD-Diffraktogramm aufgenommen, welches in Fig. 2 dargestellt ist.

### Beispiel 4

3,5-Hydrat des Calciumsalzes von *(S)*-{8-Fluor-2-[4-(3-methoxyphenyl)piperazin-1-yl]-3-[2-methoxy-5-(trifluormethyl)phenyl]-3,4-dihydrochinazolin-4-yl}essigsäure

Etwa 100 mg des in Beispiel 3 erhaltenen Salzes werden in 1 ml Ethanol Wasser 1:1 suspendiert und die erhaltene Suspension wird 1 Woche bei 25°C geschüttelt. Der erhaltene kristalline Feststoff wird abfiltriert und der erhaltene Rückstand bei Raumtemperatur und Umgebungsfeuchte getrocknet. Der erhaltene Rückstand entspricht dem 3,5-Hydrat des Calciumsalzes von *(S)*-{8-Fluor-2-[4-(3-methoxyphenyl)piperazin-1-yl]-3-[2-methoxy-5-(trifluormethyl)phenyl]-3,4-dihydrochinazolin-4-yl}essigsäure.

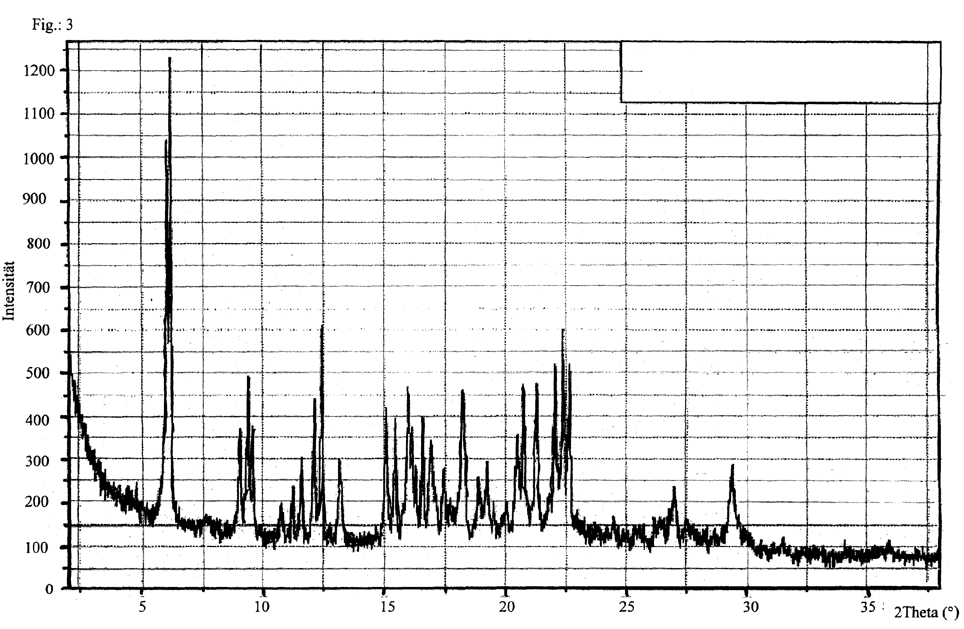

Unter Verwendung des im Beispiel 4 erhaltenen kristallinen Feststoffs wurde unter den gleichen Bedingungen wie in Beispiel 2 genannt ein Pulver-XRD-Diffraktogramm aufgezeichnet, welches in Fig. 3 dargestellt ist.

### C) Löslichkeitsbestimmungen

Zur Bestimmung der Löslichkeit wurde von dem Salz von Beispiel 2 eine gesättigte Lösung in Phosphatpuffer bei pH 7,0 angefertigt und bei Raumtemperatur 2 h geschüttelt. Die erhaltenen Suspensionen wurden über einen Spritzenfilter (0,45 µm Porendurchmesser) gefiltert und die klaren Lösungen wurden nach Verdünnen per HPLC vermessen. Als Nullprobe wurde reiner Phosphatpuffer verwendet. Die Löslichkeit wurde bezogen auf die Absorption einer Referenzlösung des amorphen Zwitterions in Phosphatpuffer berechnet. Die Ergebnisse der Messung sind nachstehend in Tabelle 4 dargestellt.

**Tabelle 4**

| Verbindung | Löslichkeit in Puffer [mg/ml] |
|---|---|
| Beispiel 6a | 0,4 (bei pH 7) |
| Beispiel 2 | > 91,7 |

### D) Bewertung der physiologischen Wirksamkeit

Die in vitro-Wirkung der erfindungsgemäßen Verbindungen auf die Replikation des HCMV (humanes Cytomegalovirus) kann in folgendem antiviralen Assay gezeigt werden: HCMV Fluoreszenzreduktionstest.

Die Testverbindungen werden als 50 millimolare (mM) Lösungen in Dimethysulfoxid (DMSO) eingesetzt. Als Referenzverbindungen können z.B. Ganciclovir®, Foscarnet® oder Cidofovir® verwendet werden. Einen Tag vor Testansatz werden 1,5 x 10⁴ humane Vorhautfibroblasten (NHDF-Zellen)/well in 200 µl Zellkulturmedium in die Wells B2-G11 von 96-well Platten (schwarz mit durchsichtigem Boden) ausgesät. Die randständigen Wells jeder 96-well Platte werden lediglich mit 200µl Medium befüllt um Randeffekte zu vermeiden. Am Versuchstag wird das Zellkulturmedium der Wells B2 - G11 jeder 96-well Platte abgesaugt und durch 100µl Virussuspension ersetzt (multiplicity of infection (MOI): 0.1-0.2). Bei dem verwendeten Virus handelt es sich um ein rekombinantes HCMV, welches eine Expressionskassette für das green fluorescence protein (GFP) in das Virusgenom integriert hat (HCMV AD 169 RV-HG(E. M. Borst, K. Wagner, A. Binz, B. Sodeik, and M. Messerle, 2008, J. Virol. 82:2065-2078.). Nach einer Inkubationszeit von 2h bei 37°C und 5% CO₂ wird das Virus-Inokulat abgesaugt und alle Wells mit Ausnahme der Wells in Spalte 3 werden mit 200µl Zellkulturmedium befüllt. Spalte 2 wird nicht weiter behandelt und dient als Viruskontrolle. Die Wells in Spalte 3 werden jeweils in Doppelbestimmung mit 300µl Testsubstanz (verdünnt in Zellkulturmedium) befüllt. Die Konzentration der jeweiligen antiviralen Substanz in Spalte 3 beträgt ∼ die 27fache Konzentration des jeweils erwarteten EC₅₀ Werts. Die Testsubstanz in Spalte 3 wird in 8 Schritten 1:3 über die 96-well Platte verdünnt indem jeweils 100µl einer Spalte in die jeweils rechte Spalte transferiert- und dort mit den vorhandenen 200µl Zellkulturmedium gemischt werden. Auf diese Weise werden drei antivirale Substanzen in Doppelbestimmungen getestet. Die Platten werden 7 Tage bei 37°C / 5% CO₂ inkubiert. Im Anschluss werden alle Wells einer Platte 3 x mit PBS (Phosphate Buffered Saline) gewaschen und mit 50 µl PBS befüllt. Danach wird die GFP-Intensität jedes Wells einer 96-well Platte mittels eines Fluoreszenzlesegerätes (FluoBox; Bayer Technology Services GmbH; Filtereinstellungen: GFP, Ex 480nm, Em 520nm) bestimmt. Aus den so erhaltenen Messwerten kann der EC₅₀ einer anti-HCMV Substanz ermittelt werden:
EC₅₀ (GFP-RA) = Substanzkonzentration in µM welche im Vergleich zur unbehandelten Viruskontrolle die GFP-Fluoreszenz in infizierten Zellen um 50% reduziert.

Repräsentative in-vitro-Wirkdaten für die erfindungsgemäßen Verbindungen sind in Tabelle 5 wiedergegeben:

**Tabelle 5**

| **Virus- Stamm** | **Beispiel 6A EC₅₀ [µM]** | **Beispiel 2 EC₅₀ [µM]** | **Ganciclovir EC₅₀ [µM]** |
|---|---|---|---|
| AD169 RV- HG | 0.0034 | 0.0039 | 2.2 |

### E) Pharmazeutische Zubereitunigen

Die erfindungsgemäßen Verbindungen können folgendermaßen in pharmazeutische Zubereitungen überführt werden:

### i.v. Lösung:

Zur Herstellung einer ersten Stammlösung werden 1,0 g des Salzes von Beispiel 2 in 10 ml Wasser für Injektionszwecke gelöst und es wird gerührt bis eine klare Lösung erhalten wird. Diese Lösung wird langsam zu einer 20 mM Phosphat-Puffer-Lösung zugegeben um Lösungen zur intravenösen Verabreichung mit Konzentrationen von 5 mg/ml bzw. 10 mg/ml herzustellen. Die pH-Werte der jeweiligen Lösungen lagen bei etwa pH 7,6 (5 mg/ml) und etwa pH 7,7 (10 mg/ml). Zum Abschluss werden die erhaltenen Lösungen sterilfiltriert und in sterilisierte geeignete Behälter abgefüllt. Die Behälter werden mit Infusionsstopfen und Bördelkappen verschlossen.

Ggf. können die so hergestellten Lösungen zur Lagerung vor dem Verschließen lyophilisiert und später zur Verwendung rekonstituiert werden.

### Tablette:

Zur Herstellung einer oralen festen Formulierung wird das Salz aus Beispiel 2 (50%) mit Calciumhydrogenphosphatdihydrat (48%), Natriumcroscarmellose (5%), Polyvinylpyrrolidon (5%) und kolloidalem Kieselgel (1%) gesiebt und gemischt. Anschließend wird gesiebtes Magnesiumstearat (1%) dazugegeben. Diese Pressmischung wird dann direkt tablettiert.

### F) Reinheit

S(+)- {8-Fluor-2-[4-(3-methoxyphenyl)piperazin-1-yl]-3-[2-methoxy-5-(trifluormethyl)-phenyl]-3,4-dihydrochinazolin-4-yl}essigsäure Natrium-Salz:

### Durchführung

313,1 g S(+)- {8-Fluor-2-[4-(3-methoxyphenyl)piperazin-1-yl]-3-[2-methoxy-5-(trifluormethyl)phenyl]-3,4-dihydrochinazolin-4-yl}essigsäure wurden in 2000 ml Planschliff-Kolben vorgelegt. Daraufhin wurden 1300 ml einer Mischung aus EtOH (vergällt)/Diisopropylether (1:1) als auch 21,9 g NaOH-Plätzchen zugegeben. Mittels Innentemperatursteuerung wurde die Mischung auf eine Innentemperatur von 50°C (Tm=51°C) für 25 Minuten erwärmt. Die NaOH Plätzchen lösen sich auf; es entsteht eine klare orangefarbene Lösung. Die Mischung wurde drei Stunden nachgerührt, so entsteht eine Suspension. Innerhalb von darauf folgenden 10 Stunden wurde die Mischung mit einer Abkühlrate von 3°C pro Stunde auf eine Innentemperatur von ca. 20°C abgekühlt und für weitere fünf Stunden bei dieser Temperatur nachgerührt. So entsteht eine dickere Suspension mit einem Gesamtvolumen von 1500 ml in einem Planschliff-Kolben, davon sind 400 ml Kristallvolumen. Unter Vakuum wird das Gesamtvolumen bei 110 bis 90 mbar auf ca. 750 ml reduziert; Innentemperatur 17-25°C, Manteltemperatur 35-50°C, Destillat: 273 g. Dies wurde ca. zwei Stunden nachgerührt. Innerhalb von 10 Minuten werden 250 ml Diisopropylether zudosiert und zwei Stunden nachgerührt. Das auf diese Weise erhaltene Produkt wird isoliert und zweimal mit je 250 ml Diisopropylether nachgewaschen; Feuchtausbeute: 284,8 g; M/W: 805,4 g. Das erhaltene Produkt wurde im VTS bei 50°C unter Stickstoff-Beiluft bei ca. 160 mbar für 20 Stunden getrocknet; Auswaage 279,4 g. Dieses Produkt wurde der folgenden Analytik unterzogen.

### Analytik

### Bestimmung des Natriumgehalts

200 mg Testsubstanz (S(+)-{8-Fluor-2-[4-(3-methoxyphenyl)piperazin-1-yl]-3-[2-methoxy-5-(trifluormethyl)phenyl]-3,4-dihydrochinazolin-4-yl}essigsäure Natrium-Salz) werden üblicherweise mit HNO₃ im Überschuss versetzt und in einem mikrowellengestützten Druckaufschluss aufgeschlossen (nach Methode 2011-0606601 Currenta, Leverkusen). Die Lösung wird mittels Flammen-Atomabsorptionsspektroskopie auf den Natriumgehalt hin untersucht (nach Methode 2011-0267201 Currenta, Leverkusen).
Ergebnis: S(+)-{8-Fluor-2-[4-(3-methoxyphenyl)piperazin-1-yl]-3-[2-methoxy-5-(trifluormethyl)phenyl]-3,4-dihydrochinazolin-4-yl}essigsäure Natrium-Salz: 3,5 Gew% Natrium
Modifikation X-Ray: kristallin
Restlösungsmittelbestimmung: 0,03 Gew% Diisopropylether

### HPLC-Analytik

Untersuchung folgender spezifischer und nichtspezifischer Verunreinigungen von_S(+)-{8-Fluor-2-[4-(3-methoxyphenyl)piperazin-1-yl]-3-[2-methoxy-5-(trifluormethyl)phenyl]-3,4-dihydrochinazolin-4-yl}essigsäure Natrium-Salz:
- Chinazolylpiperazin
- Di-p-toluoylweinsäure
- Chinazolyldipiperazin
- Chinazolinethylester
- einzelne nichtspezifische Verunreinigungen
mittels Hochdruckflüssigkeitschromatographie (HPLC);
Reversed-Phase Methode;
Detektion: UV-Bereich;
Analyse: Flächenprozent-Methode mit Flächenkorrekturfaktoren

### Geräte

1. Hochdruckflüssigkeitschromatograph mit thermostatisiertem Säulenofen, UV-Detektor und Datenauswertesystem
2. Metallsäule aus Edelstahl

| | |
|---|---|
| Länge: | 15 cm |
| Innendurchmesser: | 3,0 mm |
| Füllung: | Prodigy ODS III, 3 µm |

### Reagenzien

1. Acetonitril, für die HPLC
2. Kaliumdihydrogenphosphat, p.a.
3. o-Phosphorsäure 85%ig, p.a.

### Prüflösung

ca. 22 mg Probe S(+)-{8-Fluor-2-[4-(3-methoxyphenyl)piperazin-1-yl]-3-[2-methoxy-5-(trifluormethyl)phenyl]-3,4-dihydrochinazolin-4-yl}essigsäure Natrium-Salz; genau gewogen, in Acetonitril zu 50,0 ml gelöst.

### Kalibrierlösung

ca. 22 mg Referenzstandard (S(+)-{8-Fluor-2-[4-(3-methoxyphenyl)piperazin-1-yl]-3-[2-methaxy-5-(trifluormethyl)phenyl]-3,4-dihydrochinazolin-4-yl}essigsäure), genau gewogen, in Acetonitril zu 50,0 ml gelöst.

### Vergleichslösung

Es wird eine Vergleichslösung analog zur Kalibrierlösung hergestellt, die zusätzlich die organischen Verunreinigungen (Chinazolylpiperazin, Di-p-toluoylweinsäure, Chinazolyldipiperazin, Chinazolinethylester) in geringer Menge enthält.

### HPLC-bedingunge

Die angegebenen Bedingungen gelten als Richtwerte und können gegebenenfalls zur Erzielung optimaler Trennungen den technischen Möglichkeiten des Chromatographen und den Eigenschaften der jeweiligen Säule angepasst werden.

### Elutionsmittel

A. 1,36 g Kaliumdihydrogenphosphat und 0,7 ml o-Phosphorsäure 85%ig mit Wasser zu 1000,0 ml lösen
B. Acetonitril

### Durchflussrate

0,5 ml/min

### Temperatur des Säulenofens

55 °C

### Detektion

| | |
|---|---|
| Messwellenlänge: | 210 nm |
| Bandbreite: | 4 nm |

### Injektionsvolumen

3 µl

### Equilibrierungszeit

10 min (unter Startbedingungen)

### Gradient

| zeit [min] | % A | % B |
|---|---|---|
| 0,00 | 80 | 20 |
| 40,00 | 55 | 45 |
| 50,00 | 20 | 80 |
| 65,00 | 20 | 80 |

### Laufzeit des Chromatogramms

### 65 min

### Präzision

Die relative Standardabweichung der Flächen von 6 Einspritzungen des Referenzstandards muss ≤ 1,5 % sein.

### Durchführung

Prüflösung, Kalibrierlösung und Vergleichslösung unter den angegebenen Bedingungen chromatographieren.

| | RT [min] | RRT | RF |
|---|---|---|---|
| BAY 73-6327 | ca. 27,9 | 1,00 | 1,00 |
| Di-p-toluoylweinsäure | ca. 30,5 | 1,09 | 1,94 |
| Chinazolylpiperazin | ca. 34,4 | 1,24 | 1,02 |
| Chinazolinethylester | ca. 38,1 | 1,37 | 1,05 |
| Chinazolyldipiperazin | ca. 42,9 | 1,54 | 0,81 |

### Auswertung

Elektronische Integration der Peakflächen.

### Berechnung des Gehaltes der org. Verunreinigungen (HPLC)

Flächenprozent-Methode mit Flächenkorrekturfaktoren (RF) soweit vorhanden.

### Ergebnis

S(+)-{8-Fluor-2-[4-(3-methoxyphenyl)piperazin-1-yl]-3-[2-methoxy-5-(trifluormethyl)phenyl]-3,4-dihydrochinazolin-4-yl}essigsäure Natrium-Salz:
- 99,9% F1%
- 0,0 Fl% für bekannte organische Verunreinigungen
- 0,07 Fl% größte nichtspezifische Verunreinigungen
- 0,0907 F1% größte einzelne nichtspezifische Nebenkomponente
- 88,0 Gew%

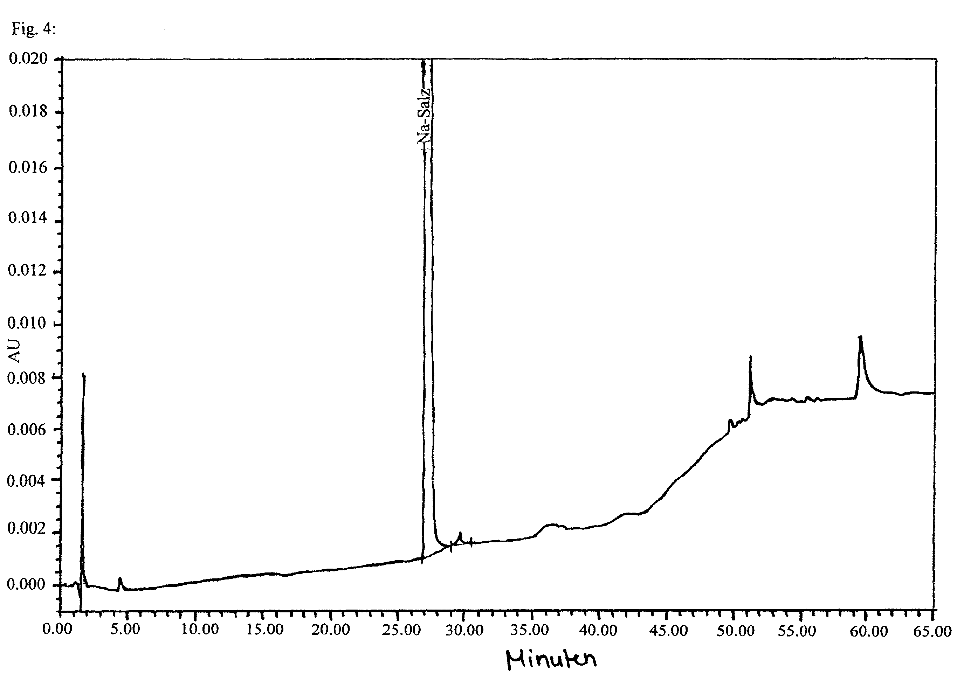

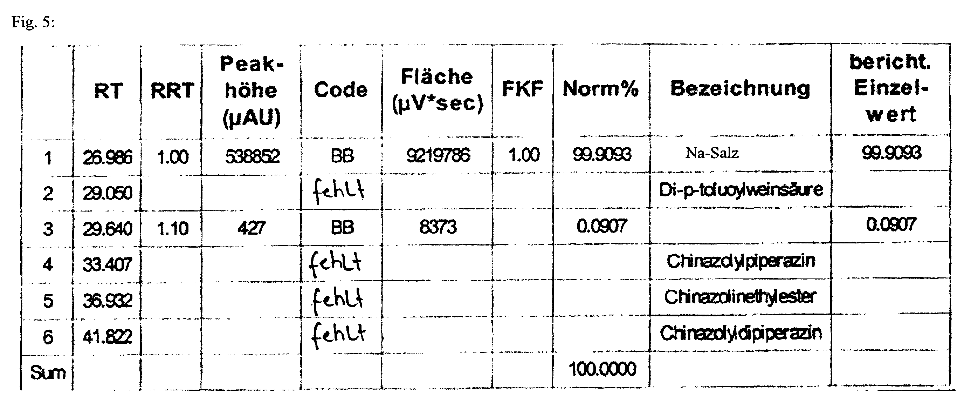

Für HPLC-Chromatogramm und dessen Analyse siehe Figuren 4 und 5.

## Patentansprüche

1. Salz von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Solvat davon, ausgewählt aus der Gruppe bestehend aus den kristallinen Natriumsalzen von {8-Fluor-2-[4-(3-methoxyphenyl)piperazin-1-yl]-3-[2-methoxy-5-(trifluormethyl)phenyl]-3,4-dihydrochinazolin-4-yl}essigsäure, den kristallinen Calciumsalzen von {8-Fluor-2-[4-(3-methoxyphenyl)piperazin-1-yl]-3-[2-methoxy-5-(trifluormethyl)phenyl]-3,4-dihydrochinazolin-4-yl}essigsäure und deren Solvaten.

2. Salz nach Anspruch 1, wobei es sich um das Monocalciumsalz von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Solvat davon handelt.

3. Salz nach Anspruch 2, wobei es sich um das 2,5-Hydrat des Monocalciumsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure handelt.

4. Salz nach Anspruch 3, **dadurch gekennzeichnet, dass** es im Pulver-XRD-Diffraktogramm charakteristische Peaks bei etwa 6.1, 9.2 und 15.5 Grad 2Theta zeigt.

5. Salz nach Anspruch 2, wobei es sich um das 3,5-Hydrat des Monocalciumsalzes von {8-Fluor-2-[4-(3-methoxyphenyl)piperazin-1-yl]-3-[2-methoxy-5-(trifluormethyl)phenyl]-3,4-dihydrochinazolin-4-yl}essigsäure handelt.

6. Salz nach Anspruch 5, **dadurch gekennzeichnet, dass** es im Pulver-XRD-Diffraktogramm charakteristische Peaks bei etwa 6.2, 12.4 und 22.4 Grad 2Theta zeigt.

7. Salz nach Anspruch 1, wobei es sich um das Mononatriumsalz von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Solvat davon handelt.

8. Salz nach Anspruch 7, wobei es sich um das 3-Hydrat des Mononatriumsalzes von {8-Fluor-2-[4-(3-methoxyphenyl)piperazin-1-yl]-3-[2-methoxy-5-(trifluormethyl)phenyl]-3,4-dihydrochinazolin-4-yl}Essigsäure handelt.

9. Salz nach Anspruch 8, **dadurch gekennzeichnet, dass** es im Pulver-XRD-Diffraktogramm charakteristische Peaks bei etwa 6.2, 20.9 und 22.4 Grad 2Theta zeigt.

10. Verfahren zur Herstellung eines Salzes nach einem der Ansprüche 1 bis 9 mit den folgenden Schritten:
a.) Lösen von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder eines Solvats davon in einer Mischung aus zumindest einem (C₁-C4)-Dialkylether und zumindest einem (C₁-C4)-Alkohol, ggf. unter Erwärmen.
b.) Zugabe von NaOH oder Ca(OH)₂ zu der in Schritt a.) erhaltenen Lösung,
c.) Entfernen eines Teil des Lösungsmittels aus der in Schritt b.) erhaltenen Lösung und ggf. Animpfen mit geeigneten Impfkristallen um die Kristallisation des Salzes bzw. eines Solvats eines Salzes auszulösen,
d.) Abtrennen des in Schritt c.) auskristallisierten Salzes oder Solvats davon,
e.) ggf. Rühren des in Schritt d.) erhaltenen Salzes oder Solvats in einem geeigneten Lösungsmittel, vorzugsweise in einer Mischung aus Wasser und zumindest einem (C₁-C₄)-Alkohol um ein gewünschtes Solvat zu erhalten, und
f.) Trocknen des in Schritt d.) oder e.) erhaltenen Salzes oder Solvats.

11. Salz nach einem der Ansprüche 1 bis 9 zur Verwendung in einem Verfahren zur Behandlung und/oder Prophylaxe von Krankheiten, insbesondere von Virusinfektionen, vorzugsweise von Infektionen mit HCMV oder einem anderen Vertreter der Gruppe der Herpes viridae.

12. Arzneimittel enthaltend ein Salz nach einem der Ansprüche 1 bis 9 in Kombination mit zumindest einem pharmazeutisch annehmbaren Hilfsstoff.

13. Arzneimittel nach Anspruch 12 zur Verwendung in einem Verfahren zur Behandlung und/oder Prophylaxe von Virusinfektionen, vorzugsweise von Infektionen mit HCMV oder einem anderen Vertreter der Gruppe der Herpes viridae.

14. Salz nach einem der Ansprüche 1 bis 9 zur Verwendung als Arzneimittel, insbesondere als Arzneimittel zur Behandlung und/oder Prophylaxe von Krankheiten, insbesondere von Virusinfektionen vorzugsweise von Infektionen mit HCMV oder einem anderen Vertreter der Gruppe der Herpes viridae.

15. Verwendung eines Salzes nach einem der Ansprüche 1 bis 9 zur Herstellung eines Medikaments zur Behandlung und/oder Prophylaxe von Virusinfektionen vorzugsweise von Infektionen mit HCMV oder einem anderen Vertreter der Gruppe der Herpes viridae.

16. Salz nach einem der Ansprüche 1 bis 9 oder Arzneimittel nach Anspruch 12 oder 13 zur Verwendung in der Bekämpfung von Virusinfektionen, vorzugsweise von Infektionen mit HCMV oder einem anderen Vertreter der Gruppe der Herpes viridae bei einem Menschen und einem Tier der (das) eine solche Behandlung benötigt.

## Claims

1. A salt of {8-fluoro-2-[4-(3-methoxyphenyl)piperazine-1-yl]-3-[2-methoxy-5-(trifluoromethyl)phenyl]-3,4-dihydroquinazoline-4-yl}acetic acid or a solvate thereof selected from the group consisting of the crystalline sodium salts of {8-fluoro-2-[4-(3-methoxyphenyl)piperazine-1-yl]-3-[2-methoxy-5-(trifluoromethyl)phenyl]-3,4-dihydroquinazoline-4-yl}acetic acid, the crystalline calcium salts of {8-fluoro-2-[4-(3-methoxyphenyl)piperazine-1-yl]-3-[2-methoxy-5-(trifluoromethyl)phenyl]-3,4-dihydroquinazoline-4-yl}acetic acid and solvates thereof.

2. The salt according to Claim 1, which is the monocalcium salt of {8-fluoro-2-[4-(3-methoxyphenyl)piperazine-1-yl]-3-[2-methoxy-5-(trifluoromethyl)phenyl]-3,4-dihydroquinazoline-4-yl}acetic acid or a solvate thereof.

3. The salt according to Claim 2, which is the 2.5-hydrate of the monocalcium salt of {8-fluoro-2-[4-(3-methoxyphenyl)piperazine-1-yl]-3-[2-methoxy-5-(trifluoromethyl)phenyl]-3,4-dihydroquinazoline-4-yl}acetic acid.

4. The salt according to Claim 3, **characterized in that** it shows characteristic peaks at about 6.1, 9.2 and 15.5 degrees 2theta in the X-ray powder diffractogram (XRD).

5. The salt according to Claim 2, which is the 3.5-hydrate of the monocalcium salt of {8-fluoro-2-[4-(3-methoxyphenyl)piperazine-1-yl]-3-[2-methoxy-5-(trifluoromethyl)phenyl]-3,4-dihydroquinazoline-4-yl}acetic acid.

6. The salt according to Claim 5, **characterized in that** it shows characteristic peaks at about 6.2, 12.4 and 22.4 degrees 2theta in the X-ray powder diffractogram (XRD).

7. The salt according to Claim 1, which is the monosodium salt of {8-fluoro-2-[4-(3-methoxyphenyl)piperazine-1-yl]-3-[2-methoxy-5-(trifluoromethyl)phenyl]-3,4-dihydroquinazoline-4-yl}acetic acid or a solvate thereof.

8. The salt according to Claim 7, which is the 3-hydrate of the monosodium salt of {8-fluoro-2-[4-(3-methoxyphenyl)piperazine-1-yl]-3-[2-methoxy-5-(trifluoromethyl)phenyl]-3,4-dihydroquinazoline-4-yl}acetic acid.

9. The salt according to Claim 8, **characterized in that** it shows characteristic peaks at about 6.2, 20.9 and 22.4 degrees 2theta in the X-ray powder diffractogram (XRD).

10. A method for producing a salt according to any of Claims 1 to 9 using the following steps:
a.) Dissolve {8-fluoro-2-[4-(3-methoxyphenyl)piperazine-1-yl]-3-[2-methoxy-5-(trifluoromethyl)phenyl]-3,4-dihydroquinazoline-4-yl}acetic acid or a solvate thereof in a mixture of at least one (C₁-C₄) dialkyl ether and at least one (C₁-C₄) alcohol, if necessary under heat.
b.) Add NaOH or Ca(OH)₂ to the solution obtained in step a.),
c.) Remove a portion of the solvent from the solution obtained in step b.) and inoculate, if necessary, with suitable seed crystals in order to initiate the crystallization of the salt or of a solvate of a salt,
d.) Separate the crystallized-out salt or solvate thereof obtained in step c.),
e.) If necessary, stir the salt or solvate obtained in step d.) in a suitable solvent, preferably in a mixture of water and at least one (C₁-C₄) alcohol in order to obtain a desired solvate, and
f.) Dry the salt or solvate obtained in step d.) or e.).

11. The salt according to any of Claims 1 to 9 for use in a method of treatment and/or prophylaxis of diseases, in particular of virus infections, preferably human cytomegalovirus (HCMV) infections or infections with another member of the herpes viridae group.

12. A drug containing a salt according to any of Claims 1 to 9 in combination with at least one pharmaceutically acceptable excipient.

13. The drug according to Claim 12 for use in a method of treatment and/or prophylaxis of virus infections, preferably human cytomegalovirus (HCMV) infections or infections with another member of the herpes viridae group.

14. The salt according to any of Claims 1 to 9 for use as a drug, in particular as a drug in a method of treatment and/or prophylaxis of diseases, in particular of virus infections, preferably human cytomegalovirus (HCMV) infections or infections with another member of the herpes viridae group.

15. The use of a salt according to any of Claims 1 to 9 for manufacturing a medicament for the treatment and/or prophylaxis of virus infections, preferably human cytomegalovirus (HCMV) infections or infections with another member of the herpes viridae group.

16. The salt according to Claims 1 to 9 or the drug according to Claim 12 or 13 for use in combating virus infections, preferably human cytomegalovirus (HCMV) infections or infections with another member of the herpes viridae group in a human and an animal who or which requires such a treatment.

## Revendications

1. Sel de l'acide acétique de {8-fluoro-2-[4-(3-méthoxyphényle)piperazine-1-yl]-3-[2-méthoxy-5-(trifluorométhyle)phényle]-3,4-dihydroquinazoline-4-yl} ou solvate de celui-ci choisi parmi le groupe comprenant les sels cristallins de sodium de l'acide acétique de {8-fluoro-2-[4-(3-méthoxyphényle)pipérazine-1-yl]-3-[2-méthoxy-5-(trifluorométhyle)phényle-3,4-dihydroquinazoline-4-yl}, les sels cristallins de sodium de l'acide acétique de {8-fluoro-2-[4-(3-méthoxyphényle)pipérazine-1-yl]-3-[2-méthoxy-5-(trifluorométhyl)phényle]-3,4-dihydroquinazoline-4-yl} et les solvates de ceux-ci.

2. Sel selon la revendication 1, qui est le sel monocalcique de l'acide acétique de {8-fluoro-2-[4-(3-méthoxyphényle)pipérazine-1-yl]-3-[2-méthoxy-5-(trifluorométhyle)phényle]-3,4-dihydroquinazoline-4-yl} ou un solvate de celui-ci.

3. Sel selon la revendication 2, qui est le 2.5-hydrate du sel monocalcique de l'acide acétique {8-fluoro-2-[4-(3-méthoxyphényle)pipérazine-1-yl]-3-[2-méthoxy-5-(trifluorométhyle)phényle]-3,4-dihydroquinazoline-4-yl}.

4. Sel selon la revendication 3, **caractérisé en ce qu'**il présente des pics caractéristiques d'environ 6.1, 9.2 et 15.5 degrés 2thêta dans le diffractogramme de poudre par rayons X (XRD).

5. Sel selon la revendication 2, qui est le 3.5-hydrate du sel monocalcique de l'acide acétique de {8-fluoro-2-[4-(3-méthoxyphényle)pipérazine-1-yl]-3-[2-méthoxy-5-(trifluorométhyle)phényle]-3,4-dihydroquinazoline-4-yl}.

6. Sel selon la revendication 5, **caractérisé en ce qu'**il présente des pics caractéristiques d'environ 6.1, 12.4 et 22.4 degrés 2thêta dans le diffractogramme de poudre par rayons X (XRD).

7. Sel selon la revendication 1, qui est le sel monosodique de l'acide acétique de {8-fluoro-2-[4-(3-méthoxyphényle)pipérazine-1-yl] -3-[2-méthoxy-5-(trifluorométhyle)phényle]-3,4-dihydroquinazoline-4-yl} ou un solvate de celui-ci.

8. Sel selon la revendication 7, qui est le 3-hydrate du sel monosodique de l'acide acétique {8-fluoro-2-[4-(3-méthoxyphényle)pipérazine-1-yl]-3-[2-méthoxy-5-(trifluorométhyle)phényle]-3,4-dihydroquinazoline-4-yl},

9. Sel selon la revendication 8, **caractérisé en ce qu'**il présente des pics caractéristiques d'environ 6.2, 20.9 et 22.4 degrés 2thêta dans le diffractogramme de poudre par rayons X (XRD).

10. Procédé de production d'un sel selon l'une quelconque des revendications 1 à 9, consistant à:
a.) Dissoudre l'acide acétique de {8-fluoro-2-[4-(3-méthoxyphényle)pipérazine-1 -yl]-3-[2-méthoxy- 5 -(trifluorométhyle)phényle] -3,4-dihydroquinazoline-4-yl} ou un solvate de celui-ci dans un mélange d'au moins un dialkyléther (C₁-C₄) et au moins un alcool (C₁-C₄), si nécessaire, sous apport de chaleur,
b.) Ajouter NaOH ou Ca(OH)₂ à la solution obtenue à l'étape a.);
c.) Enlever une partie du solvant de la solution obtenue à l'étape b.) et l'inoculer, si nécessaire, avec des cristaux adaptés pour lancer la cristallisation du sel ou d'un solvate d'un sel;
d.) Séparer le sel cristallisé ou le solvate de celui-ci obtenu à l'étape c.);
e.) Si nécessaire, remuer le sel ou le solvate obtenu à l'étape d.) dans un solvant adapté, de préférence dans un mélange d'eau et d'au moins un alcool (C₁-C₄) pour obtenir un solvate désiré; et
f.) Sécher le sel ou le solvate obtenu à l'étape c.), d.) ou e.).

11. Sel selon l'une quelconque des revendications 1 à 9, destiné à être utilisé dans une méthode de traitement et/ou de prévention de maladies, en particulier les infections virales, de préférence les infections au cytomégalovirus humain (HCMV) ou les infections par un autre membre de la famille des Herpes viridae.

12. Médicament contenant un sel selon l'une quelconque des revendications 1 à 9 en association avec au moins un excipient pharmaceutiquement acceptable.

13. Médicament selon la revendication 12, destiné à être utilisé dans une méthode de traitement et/ou de prévention d'infections virales, de préférence les infections au cytomégalovirus humain (HCMV) ou les infections par un autre membre de la famille des Herpes viridae.

14. Sel selon l'une quelconque des revendications 1 à 9, destiné à être utilisé en tant que médicament, en particulier comme médicament dans une méthode de traitement et/ou de prévention de maladies, en particulier les infections virales, de préférence les infections au cytomégalovirus humain (HCMV) ou les infections par un autre membre de la famille des Herpes viridae.

15. Utilisation d'un sel selon l'une quelconque des revendications 1 à 9, pour la fabrication d'un médicament destiné au traitement et/ou à la prévention d'infections virales, de préférence les infections au cytomégalovirus humain (HCMV) ou les infections par un autre membre de la famille des Herpes viridae.

16. Sel selon les revendications 1 à 9 ou médicament selon la revendication 12 ou 13, destiné à être utilisé dans la lutte contre les infections virales, de préférence les infections au cytomégalovirus humain (HCMV) ou les infections par un autre membre de la famille des Herpes viridae chez un être humain ou un animal qui a besoin d'un tel traitement.
